# EUROPEAN PATENT SPECIFICATION

(11) **EP 1 441 844 B1**
(45) Date of publication and mention of the grant of the patent: **19.01.2005**
(21) Application number: 02800606.2
(22) Date of filing: 08.10.2002
(51) Int. Cl.: B01F 17/00

(54) **HYDROPHOBICALLY MODIFIED SACCHARIDE SURFACTANTS**
HYDROPHOBISCH MODIFIZIERTE SACCHARID-TENSIDE
TENSIOACTIFS A BASE DE SACCHARIDES RENDUS HYDROPHOBES PAR MODIFICATION

(30) Priority: 09.10.2001 EP 01124037
(43) Date of publication of application: 04.08.2004
(73) Proprietor: Tiense Suikerraffinaderij N.V., 1150 Brussel (BE)
(72) Inventor: BOOTEN, Karl, B-3450 Geetbets (BE); LEVECKE, Bart, B-1020 Laken (BE); STEVENS, Christian, Victor, B-9820 Merelbeke-Schelderode (BE)
(74) Representative: Hermans, Johny
(86) International application number: PCT/EP2002/011233
(87) International publication number: WO 2003/031043

(56) References cited:
- EP-A- 0 964 054
- US-A- 5 502 180
- US-A- 5 877 144

## Description

### Field of the invention

The present invention relates to the use as surfactant of hydrophobically modified saccharides for the preparation of dispersions of multiphase systems composed of one or more liquids, solids and/ or gases dispersed in a continuous aqueous phase containing an electrolyte, to said dispersions, as well as to a method for preparing and stabilising dispersions.

### Background and prior art

Industry is often confronted with the technical problem of making dispersions from a mixture of two or more phases which are non-miscible or only partly miscible with each other. The term dispersion refers to a composition that consists of a continuous phase that contains dispersed in it small particles of one or more other phases forming one or more discontinuous phases. The dispersions which are most frequently encountered and, accordingly, which are of high interest to industry, are composed of a continuous aqueous phase and one or more discontinuous non-aqueous phases.

The term dispersion refers hereinafter to compositions that consist of a continuous aqueous phase that contains dispersed in it small particles of one or more other phases forming one or more discontinuous phases (also named dispersed phases). Depending on the nature of the other phase(s) involved, the particles can be droplets (in case of a liquid phase), solid particles (in case of a solid phase) or gas bubbles (in case of a gaseous phase). Dispersions are commonly prepared from a mixture or a pre-mix of the composing phases by thoroughly mixing the phases, for example by means of a high speed mixer or a homogeniser in case of liquid phases, or through grinding by means of a bead mill or a colloid mill in case of the presence of a solid phase. However, due to the non-miscibility or partial miscibility of the composing phases, the obtained dispersions are commonly unstable.

In systems with a discontinuous liquid non-aqueous phase, the instability is characterised by the coalescence of the droplets of the dispersed liquid phase. In systems with a discontinuous solid phase, the instability is characterised by the flocculation, typically with formation of aggregates or clumps, of the dispersed solid phase. In systems with a discontinuous gaseous phase, commonly named foams, the instability is characterised by fusing of the gas bubbles, resulting in the collapse of the foam.

As a result of said coalescence, flocculation or collapse of, respectively, the droplets, the solid particles or the gas bubbles, the dispersion may separate to a more or lesser extent into separate phases, and may ultimately separate completely into separate phases, which is thermodynamically the most favourable system.

It is already known for a long time that the addition of certain compounds to a mixture of non-miscible or partly miscible phases enables or facilitates the formation of dispersions and/or improves the stability of said dispersions against coalescence, flocculation and/or collapse, hereinafter termed in short stability. As a result of said improved stability, the coalescence, flocculation and/ or collapse of the discontinuous phase or phases is inhibited, delayed or reduced to a more or lesser extent, compared to those of dispersions prepared in the absence of said compounds.

Typically said compounds are molecules that consist of a hydrophilic moiety that interacts with the aqueous continuous phase, and a hydrophobic moiety that interacts with the non-aqueous phase. They usually reduce the interfacial tension between liquid phases, solid/liquid phases and/or gas/liquid phases and, accordingly, they are said to present tensio-active properties. Said reduction facilitates the dispersion in the continuous aqueous phase of a liquid or of aggregates of liquid or solid particles into single particles, improves the wettability of a solid phase by a liquid phase, and enables the formation of a foam. As a result thereof the stability of the dispersions is improved and the tendency of the dispersions to separate into separate phases is reduced.

The compounds which enable or facilitate the formation of a dispersion and/ or improve the stability of a dispersion against coalescence, flocculation and/ or collapse are commonly referred to as surfactants, tensio-active agents or surface active agents. After the discovery of the effect of the tensio-active agents on dispersions, it has become common practice to add a surfactant to one or more of the phases or to a mixture of the phases of a composition to enable or facilitate the preparation of a dispersion from said phases and/or to improve the stability of the dispersion.

There exist various kinds of dispersions, which are in fact multiphase systems, such as biphase systems and triphase systems in which two or more phases appear as a discontinuous phase in the form of very small liquid, solid or gaseous particles, dispersed in a continuous aqueous phase. Biphase systems include systems composed of a gas phase (gas bubbles) / continuous aqueous phase; a liquid phase (droplets)/continuous aqueous phase; or a solid phase (solid particles)/ continuous aqueous phase. Triphase systems include systems composed of a gas phase/ liquid phase/continuous aqueous phase; a gas phase/ solid phase/ continuous aqueous phase; or a solid phase/ liquid phase/ continuous aqueous phase.

The dispersions of multiphase systems are conventionally classified, in function of the nature of the composing phases, in four groups, as follows:
(i) suspensions: systems consisting of a discontinuous solid phase which is composed of one or more solid compounds in a finely divided form, dispersed in a continuous aqueous phase;
(ii) emulsions: systems consisting of a discontinuous liquid phase in a finely divided form, which is composed of one or more miscible, partly miscible or non-miscible liquids, dispersed in a continuous aqueous phase;
(iii) foams: in biphase systems: consisting of a discontinuous gas phase composed of bubbles of a gas or mixture of gases, dispersed in a continuous aqueous phase, and, in triphase systems: consisting of a discontinuous gas phase composed of bubbles of a gas or mixture of gases, dispersed in a said suspension or in a said emulsion;
(iv) suspoemulsions: triphase systems consisting of a discontinuous solid phase composed of finely divided particles of one or more solids and a discontinuous liquid phase composed of one or more miscible, partly miscible or non-miscible liquids, dispersed in a continuous aqueous phase.

Furthermore, still other variations of multiphase systems exist, for example a system consisting of a gas phase, a solid phase and two liquid phases.

All said multiphase systems are embraced herein by the term dispersion.

Surface active agents are usually classified, based on their action on the phases of a dispersion as *i.a.* detergent, emulsifier, emulsion stabiliser, wetting agent, suspension stabiliser, foaming agent, or foam stabiliser.

The action and effect of the surfactant largely depend of its chemical structure and/or the nature of the components of the dispersion. Accordingly, for the preparation of a dispersion, the kind of surfactant is commonly selected in function of the components of the multiphase system involved. Said selection is often made by the skilled person on the basis of screening experiments that are carried out routinely.

The earliest surfactants, typically used as detergents, were alkali soaps of naturally occurring fatty acids, commonly termed soaps, such as sodium palmitate. These compounds have been mostly replaced now by more effective synthetic surfactants. Typical classes of synthetic surface-active agents that are used in industry now include anionic, cationic, amphoteric and nonionic surfactants.

Anionic surfactants include, apart from said soaps, for example alkylbenzenesulfonates (ABS). ABS-type surfactants, being poorly biodegradable, are nowadays mostly substituted for the better biodegradable linear alkylsulfonates (LAS).

Cationic surfactants typically include tetra-alkyl ammonium salts, such as dodecyl trimethyl ammonium chloride.

Amphoteric surfactants commonly include zwitterionic type compounds, such as 3-[N,N-dimethyl N-dodecyl ammonio] 1-propane sulphonate.

Non-ionic surfactants mostly belong to the class of alkoxylated compounds, typically ethoxylated compounds, such as dodecyl hexaoxyethylene glycol monoether.

The above surfactants perform satisfactorily in many multiphase systems enabling the preparation of dispersions of industrially acceptable stability against coalescence, flocculation and/or collapse.

The presence of an electrolyte in the aqueous phase of a dispersion usually destabilises the dispersion, in spite of the presence of a surfactant, and provokes a considerable up to a complete coalescence of the discontinuous liquid phase(s), flocculation of the solid phase(s), and/or collapse of the foam. Usually the higher the concentration of the electrolyte (up to a ceiling level) in the aqueous phase of a dispersion, and the higher the temperature, the more pronounced the destabilisation of the dispersion.

However, industry often has to prepare dispersions of multiphase systems, typically biphase and triphase systems, that comprise a continuous aqueous phase containing a high concentration of one or more electrolytes. In these particular multiphase systems most known surfactants fail to provide dispersions of industrially acceptable stability.

As a result thereof, industry is often confronted with the technical problem of providing dispersions of multiphase systems comprising a continuous aqueous phase containing one or more electrolytes, which present an industrially acceptable stability against coalescence, flocculation and/ or collapse, particularly when the electrolyte is present at a high concentration and/or the dispersion is at a temperature above room temperature.

### Aim of the invention.

The present invention aims to provide a solution to one or more of said technical problems as well as to other ones.

### Description of the invention

In the search for improved and/or alternative surfactants, the inventors have unexpectedly found that the use as surfactants of a particular class of hydrophobically modified saccharides at certain concentration ranges enables to solve one or more of the said technical problems.

Accordingly, in one aspect the present invention relates to a method of use as surfactant of hydrophobically modified saccharides at certain concentration ranges for the preparation of stable dispersions or dispersions of improved stability from multiphase systems that comprise a continuous aqueous phase containing a high concentration of one or more electrolytes.

In an other aspect, the present invention relates to a method for the preparation of stable dispersions or dispersions of improved stability from multiphase systems comprising a continuous aqueous phase containing a high concentration of one or more electrolytes, by using a hydrophobically modified saccharide at certain concentration ranges as surfactant.

In still a further aspect, the present invention relates to stable dispersions or dispersions of improved stability of multiphase systems that comprise a continuous aqueous phase containing a high concentration of one or more electrolytes, and a hydrophobically modified saccharide at certain concentration ranges as surfactant.

By dispersion is meant hereinafter all multiphase systems composed of at least two phases of which one phase is a continuous aqueous phase, and the other phase or phases are discontinuous phases which are in the form of very small liquid, solid and/or gaseous particles that are dispersed in the said continuous aqueous phase. Said discontinuous phases are also named dispersed phase(s). The term dispersion preferably refers to biphase systems and triphase systems and includes suspensions, emulsions, foams and suspoemulsions.

By stable dispersion is meant herein a dispersion of industrially acceptable stability, which means that within a set time period and temperature range which are suitable for the intended industrial application, (i) in case of an emulsion: the discontinuous liquid phase(s) present an industrially acceptable stability against coalescence, (ii) in case of a suspension: the solid particles of the discontinuous phase(s) present an industrially acceptable stability against flocculation, (iii) in case of a foam: the gas bubbles present an industrially acceptable stability against collapse, and (iv) in case of a suspoemulsion: any of the discontinuous phases present an industrially acceptable stability against coalescence and/ or flocculation.

By dispersion with improved stability is meant herein a dispersion that presents an improved stability against coalescence, flocculation and/or collapse, compared to dispersions known in the art.

A phenomenon often encountered with dispersions, typically in emulsions, suspensions and suspoemulsions, is that the dispersed particles of the discontinuous phase(s), being droplets and/or solid particles, upon standing, converge without coalescing or flocculating at either the upper or lower side of the continuous aqueous phase. This phenomenon is due to the difference in density between the continuous aqueous phase and the dispersed phase(s), and may even make appear a part of the continuous aqueous phase about free of dispersed particles. In case of an emulsion, this phenomenon is commonly named creaming. It is emphasised that said phenomenon is not regarded as instability and that a dispersion presenting creaming is considered herein as still a stable dispersion.

By electrolyte is meant herein a salt which dissolved in water or in contact with water or an aqueous medium will provide ionic conductivity as a result of its partial or complete dissociation into cations and anions.

The class of hydrophobically modified saccharides in accordance with the present invention consists of substituted polymeric saccharides corresponding to general formula (I) or (II)

[A] ₙ (-M) ₛ (I) [B] ₘ (-M) _{s'} (II)

wherein
[A] ₙ represents a fructan-type saccharide with [A] representing a fructosyl unit or a terminal glucosyl unit and n representing the number of fructosyl and glucosyl units in said saccharide molecule, n being named degree of polymerisation (DP),
[B] ₘ represents a starch-type saccharide with [B] representing a glucosyl unit and m representing the number of glucosyl units in said saccharide molecule, m being named degree of polymerisation (DP),
(-M) represents a hydrophobic moiety that substitutes a hydrogen atom of a hydroxyl group of said fructosyl or glucosyl units, said moiety being selected from the group consisting of an alkylcarbamoyl radical of formula R-NH-CO- and an alkylcarbonyl radical of formula R-CO- , wherein R represents a linear or branched, saturated or unsaturated alkyl group with 4 to 32 carbon atoms, and s and s' , which can have the same value or not, represent the number of hydrophobic moieties that substitute the fructosyl or glucosyl unit, expressed as (number) average degree of substitution (av. DS).

The substituted polymeric saccharides of formula (I) and (II) according to the present invention are derived by appropriate substitution from homodisperse or polydisperse, linear or branched fructan-type saccharides which are selected from the group consisting of inulin, oligofructose, fructo-oligosaccharide, partially hydrolysed inulin, levan, and partially hydrolysed levan, or starch-type saccharides which are selected from the group consisting of modified starches and starch hydrolysates, namely by the substitution of the hydrogen atom of one or more of the hydroxyl groups of the fructosyl and/ or glucosyl units by an hydrophobic moiety (-M), defined above.

Inulin is a fructan composed of molecules mainly consisting of fructosyl units that are bound to one another by ß (2-1) fructosyl - fructosyl bounds, and possibly having a terminal glucosyl unit It is synthesised by various plants as a reserve carbohydrate, by certain bacteria, and can also be synthetically obtained through an enzymatic process from sugars containing fructose units, such as sucrose. Very suitable in accordance with the present invention is polydisperse, linear inulin or slightly branched inulin (typically inulin having a branching that is below 20 %, preferably below 10 %) from plant origin with a degree of polymerisation (DP) ranging from 3 to about 100.

Very suitable inulin is chicory inulin that has a DP ranging from 3 to about 70 and an av. DP of ≥ 10. Even more suitable is chicory inulin that has been treated to remove most monomeric and dimeric saccharide side products, and that optionally also has been treated to remove inulin molecules with a lower DP, typically a DP from 3 to about 9.

Said grades of chicory inulin can be obtained from roots of chicory by conventional extraction, purification and fractionation techniques, as for example disclosed in US 4,285,735, in EP 0 670 850 and in EP 0 769 026. They are commercially available for example from ORAFTI, Belgium as RAFTILINE® ST (standard grade chicory inulin with av. DP of 10-13), RAFTILINE® LS (standard grade chicory inulin with an av. DP of 10-13, and with in total less than 0.5 wt% (on dry substance) of monomeric and dimeric saccharides) and RAFTILINE® HP (high performance grade chicory inulin, with an av. DP of about 23 which contains only minor amounts of monomeric saccharides, dimeric saccharides and inulin molecules with a DP from 3 to about 9).

Further suitable saccharides of the fructan-type include partially hydrolysed inulin and inulin molecules with a DP ranging from 3 to about 9, namely oligofructose and fructo-oligosaccharide (*i.e.* oligofructose molecules with an additional terminal glucosyl unit). Said saccharides are known in the art. Typically suitable products are obtained by partial, enzymatic hydrolysis of chicory inulin, for example as disclosed in EP 0 917 588. They are commercially available, for example as RAFTILOSE® P95 from ORAFTI, Belgium.

Further suitable saccharides of the fructan-type are levans and partially hydrolysed levans, molecules mainly consisting of fructosyl units that are bound to each other by β (2-6) fructosyl - fructosyl bounds and may have a terminal glucosyl unit Levans and partially hydrolysed levans are known in the art.

Modified starches and starch hydrolysates are polymeric saccharides of the starch-type, consisting of D-glucosyl units which are linked to one another. In starch the glucosyl units are typically linked by α-1,4-glucosyl-glucosyl bounds, forming linear molecules, named amylose, or by α-1,4- and α-1,6 glucosyl-glucosyl bounds, forming branched molecules, named amylopectin. Starch occurs in various plants as a reserve carbohydrate and is manufactured at industrial scale from plant sources by conventional techniques.

The linkages between the glucosyl units in starch-type molecules are sensitive to disruption. This phenomenon is industrially exploited to prepare modified starches and starch hydrolysates from starch through thermal treatment commonly in the presence of a catalyst, through acidic hydrolysis, enzymatic hydrolysis, or shearing, or through combinations of such treatments. Depending on the source of the starch and the reaction conditions, a wide variety of modified starches and starch hydrolysates can be prepared at industrial scale by conventional methods. Modified starches (commonly named dextrins) and starch hydrolysates are known in the art.

Starch hydrolysates conventionally refer to polydisperse mixtures composed of D-glucose, oligomeric (DP 2 to 10) and/or polymeric (DP>10) molecules composed of D-glucosyl chains. D-glucose (dextrose) presents strong reducing power and said oligomeric and polymeric molecules also present reducing power resulting from the presence of reducing sugar units (which are essentially terminal glucosyl units). Accordingly, starting from a given starch, the more the hydrolysis has proceeded, the more molecules (monomeric D-glucose, oligomeric and polymeric molecules) will be present in the hydrolysate, and thus the higher will be the reducing power of the hydrolysate. The reducing power has become the feature of choice of industry to differentiate the various starch hydrolysates. It is expressed in dextrose equivalent (D.E.) which formally corresponds to the grams of D-glucose (dextrose) per 100 grams of dry substance. D-glucose having per definition a D.E. of 100, the D.E. indicates the amount of D-glucose and reducing sugar units (expressed as dextrose) in a given product on dry product basis. The D.E. is in fact a measurement of the extent of the hydrolysis of the starch and also a relative indication of the average molecular weight of the starch-type saccharide molecules in the hydrolysate. Starch hydrolysates may range from a product essentially composed of glucose, over products with a D.E. greater than 20 (commonly named glucose syrups), to products with a D.E. of 20 or less (commonly named maltodextrins). Starch hydrolysates are typically defined by their D.E. value. Often industry additionally defines starch hydrolysates by the source of the starch and/or their method of manufacture.

Starch hydrolysates that are very suitable saccharides for the preparation of hydrophobically modified saccharides of formula II above, have a D.E. ranging from 2 to 47. They may be obtained by conventional processes from various starch sources, such as for example starch from corn, potato, tapioca, rice, sorghum and wheat.

Starch hydrolysates are commercially available. For example, in the brochure from Roquette company " GLUCIDEX® Brochure 8/ 09.98 ", maltodextrins and glucose syrups are described in detail and various grades are offered for sale.

In a preferred embodiment of the invention, the above defined fructan-type saccharides and starch-type saccharides are substituted by two or more alkylcarbamoyl moieties of formula R-NH-CO- in which the R group can be the same or different

In another preferred embodiment of the invention, the above defined fructan-type saccharides and starch-type saccharides are substituted by two or more alkylcarbonyl moieties of formula R-CO- in which the R group can be the same or different.

In still another preferred embodiment of the invention, the above defined fructan-type saccharides and starch-type saccharides are substituted by two or more hydrophobic moieties defined above, which are of a different nature. Accordingly, the saccharide may be substituted by one or more alkylcarbamoyl moieties and by one or more alkylcarbonyl moieties.

In said alkylcarbamoyl and alkylcarbonyl moieties, the alkyl group (R) is a linear or branched radical of 4 to 32 carbon atoms. Preferably, it is a linear radical with 6 to 20 carbon atoms, more preferably with 6 to 18 carbon atoms, most preferably with 8 to 12 carbon atoms. Said alkyl radical can be a saturated alkyl radical as well as an unsaturated alkyl radical, typically an unsaturated alkyl radical with one or two double or triple carbon-carbon bounds.

In a preferred embodiment said alkyl group (R-) is a linear, saturated or mono-unsaturated alkyl radical with 6 to 18 carbon atoms.

Highly preferred hydrophobic moieties include the ones shown in Table 1 below.

The fructosyl and glucosyl units of said polymeric saccharide molecules of the fructan-type and starch-type have two, three or four hydroxyl groups of which the hydrogen atom can be substituted by a said hydrophobic moiety, depending respectively whether the unit is at a branching point of the saccharide chain, is a unit of a linear part of the chain or is a terminal unit of the chain. The number of hydrophobic moieties per unit, indicated by the indexes s and s' in formula (I), respectively formula (II) above, is commonly expressed as the average degree of substitution (av. DS), corresponding to the average number of hydrophobic moieties per unit of the substituted saccharide molecule. The av. DS of hydrophobically substituted saccharides of formula (I) and (II) which are suitable in accordance with the present invention ranges from 0.01 to 0.5, preferably from 0.02 to 0.4, more preferably from 0.05 to 0.35, most preferably from 0.1 to 0.3.

The hydrophobically modified saccharides of formula (I) and (II) are known in the art and can be prepared by conventional methods. Hydrophobically modified saccharides of formula (I) and (II) wherein the hydrophobic moiety is an alkylcarbamoyl radical (R-NH-CO-) can be prepared for example by reaction of the appropriate fructan-type saccharide or starch-type saccharide with an alkyl isocyanate of formula R - N = C = O (R having the meanings given above) in an inert solvent as described *e.g.* in WO 99/64549 and WO 01/44303. Hydrophobically modified saccharides of formula (I) and (II) wherein the hydrophobic moiety is an alkylcarbonyl radical (R-CO-) can be prepared by conventional esterification reactions, as for example disclosed in EP 0 792 888 and EP 0 703 243, typically by reaction of the appropriate fructan-type saccharide or starch-type saccharide with an anhydride of formula R-CO-O-CO-R or an acid chloride of formula R-CO-Cl (R having the meanings given above) in an appropriate solvent. Also Japanese patent application JP 3-197409 discloses fatty acid esters of fructo-oligosaccharides of the inulin-type as well as of the levan-type.

Many of the hydrophobically modified saccharides of formula (I) and (II) are disclosed to present tensio-active properties and to be useful as surfactant for the preparation of dispersions containing a continuous aqueous phase that is free of electrolytes or that contains only low concentrations of an electrolyte.

However, the prior art is absolutely silent about the particular and unexpected tensio-active properties of the hydrophobically modified saccharides of formula (I) and (II) above which enable to use these hydrophobically modified saccharides as surfactants for the manufacture of dispersions that are stable or present improved stability from multiphase systems that comprise a continuous aqueous phase containing a high concentration of one or more electrolytes. Said electrolytes typically include metal salts, ammonium salts, amine salts, quaternary ammonium salts, salts of organic bases and mixtures thereof, which partially or completely dissociate in an aqueous medium forming cations and anions, or zwitterions. The cations include metal ions from monovalent, bivalent, trivalent and tetravalent metals, and ions involving a nitrogen atom. Typical metal cations include ions of lithium, sodium, potassium, magnesium, calcium, barium, chromium, manganese, iron, cobalt, nickel, copper, zinc and aluminium. Typical cations involving a nitrogen atom include ammonium ions, ions from salts of primary, secondary and tertiary amines such as for example monoalkyl amines, dialkyl amines, trialkyl amines and benzyl dialkyl amines, quaternary ammonium ions, and ions formed from organic nitrogen bases such as for example morpholine, piperazine and heterocyclic compounds such as *e.g.* pyridine.

Said anions include hydroxyl anions and anions derived from inorganic acids as well as from organic acids, such as, for example, hydrogen halides including hydrofluoric acid, hydrochloric acid, hydrobromic acid and hydroiodic acid, sulphuric acid, phosphoric acid, carbonic acid, formic acid, acetic acid and lactic acid.

By concentration of one or more electrolytes is meant herein the total concentration of the one or more electrolytes in the continuous aqueous phase of the dispersion. By high concentration is meant a total concentration of the one or more electrolytes in the continuous aqueous phase which is higher, typically significantly higher, than the total concentration of the electrolyte(s) in the continuous aqueous phase of dispersions disclosed in the prior art. In accordance with the present invention, said total concentration in the continuous aqueous phase ranges from the lower limit of 0.1 to 1 mole per litre, depending on the nature of the electrolyte(s), including the valency of the ions involved, and the temperature to which the dispersion is subjected, up to the higher limit of the range being the limit of the solubility of the electrolyte(s) in water at 25°C.

Typically, said high concentration ranges from about 0.5 mole to about 5 moles per litre, more typically from about 1 mole to about 5 moles per litre, even from about 2 moles to about 5 moles electrolyte(s) per litre continuous aqueous phase. The high concentration typically ranges for salts of monovalent cations from 0.1 mole, most typically from 0.5 mole, to about 5 mole per litre aqueous phase, for salts of bivalent cations from 0.1 mole, most typically from 0.5 mole, to about 3 moles per litre aqueous phase, and for salts of trivalent cations from 0.1 to about 1 mole per litre aqueous phase.

In the dispersions according to the subject invention, the ratio non-aqueous phase(s) / aqueous phase may range from about 90: 10 to about 1 : 99. Preferably said ratio ranges from about 65 : 35 to about 20 : 80. A typical ratio is 50 : 50. In case of non-aqueous liquid phase(s) or gas phases said ratio is expressed as volume : volume ratio; in case of non-aqueous solid phase(s), the ratio is expressed as weight: volume ratio.

Since the hydrophobically modified saccharides of formula (I) and (II) are more or less sensitive to hydrolysis, the pH of the aqueous phase of the multiphase system is preferably kept between 4 and 10, more preferably between 5 and 9, most preferably between 6 and 8.

The efficiency of the hydrophobically modified saccharides of formula (I) and (II) acting as surfactants in the preparation of dispersions from multiphase systems in accordance with the present invention depends from various factors. Said factors include the kind of the multiphase system, the kind and nature of the composing phases, the structure of the surfactant including the type and the degree of polymerisation of the saccharide, the nature of the hydrophobic moiety or moieties, the nature of the alkyl group of said hydrophobic moiety or moieties and the average degree of substitution DS. The efficiency furthermore depends on the nature of the electrolyte(s), the concentration of the respective electrolytes, the total concentration of the electrolyte(s) in the aqueous phase, the method of manufacture of the dispersion, the pH of the aqueous phase and the temperature at which the dispersion is stored. Usually the higher the total concentration of electrolyte(s) in the aqueous phase, the higher the amount of hydrophobically modified saccharide that is required for the preparation of a stable dispersion.

In accordance with the present invention also mixture of two or more surfactants of formula (I) and/or formula (II) may be used.

In addition to the hydrophobically modified saccharides according to the present invention, also conventional surfactants may be used to facilitate the formation of the dispersion and/or to improve its stability.

Furthermore, in cases where creaming occurs in emulsions or the comparable phenomenon in suspensions prepared in accordance with the present invention, conventional thickeners can be added to the dispersion to reduce the difference in density between the phases. As a result thereof the dispersed liquid and/ or solid phases remain better and/ or longer homogeneously dispersed in the continuous aqueous phase.

It is to be noted that the hydrophobically modified saccharides of formula (I) and (II) above or a mixture thereof also perform well as surfactants for the preparation of stable dispersions, or dispersions with improved stability, comprising an aqueous phase which is free of electrolytes or contains only low concentrations of electrolytes.

For the preparation of a dispersion in accordance with the present invention, an amount is used of surfactant or mixture of surfactants of formula (I) and/or formula (II) above, that ranges from about 0.10 to about 20 %, preferably from about 0.15 to about 15 %, more preferably from about 0.20 to about 15 %, typically from about 0.50 to about 10 %. In case of emulsions, the % is expressed as % weight/volume (%w/v) on dispersed phase(s), in case of suspensions as % weight/weight (% w/w) on dispersed phase(s), and in case of foams as % weight/volume (%w/v) on the aqueous phase.

Preferred multiphase systems in accordance with the present invention include the biphase systems: oil phase/aqueous phase *(i.e.* emulsions), solid phase/ aqueous phase *(i.e.* suspensions), and gas phase/aqueous phase *(i.e.* foams), and the triphase systems: solid phase/oil phase/aqueous phase (*i.e.* suspoemulsions), gas phase/ oil phase/ aqueous phase, and solid phase/ gas phase/aqueous phase.

The present invention is illustrated by the examples given below. The dispersions were prepared and evaluated according to the following methods.

### Example 1.

Several emulsions were prepared according to four different methods.
In a first step of these methods the oil phase was added dropwise to the aqueous phase containing the surfactant (hydrophobically modified saccharide of formula (I) or (II) in demineralised water), while the mixture was stirred by means of a high speed homogeniser (for example CAT* X620, * trade name of Ingenieurbüro CAT, M. Zipperer GmbH, Staufen, Germany).
The dispersions were prepared on a 50 ml scale.
The particular conditions of the addition of the oil phase to the aqueous phase and of the homogenising applied in each method are indicated below.
Method A (Four step process): The oil was added during the first step. In the four step mixing procedure, the mixing speed was stepwise increased as follows: 2 minutes at 9,500 rpm, followed by 1 minute at 13,500 rpm, followed by 45 seconds at 20,500 rpm and finally 1 minute at 24,000 rpm. Mixing was carried out by means of a high speed homogeniser.
Method B (One-step process): The oil was added during the first minute of the mixing process while stirring the mixture at 9,500 rpm, and this speed was maintained for 5 minutes in total. Mixing was carried out by means of a high speed homogeniser.
Method C (Two-step process): The oil was added during the first minute of the mixing process with stirring at 9,500 rpm, and this speed was maintained for 5 minutes in total. Mixing was carried out with a high speed homogeniser. Then, the obtained mixture was treated at 700 bar for 1 minute in a high pressure homogenizer (Microfluidizer®, trade name of Microfluidics Corp., USA).
Method D (Two-step process): The oil was added during the first minute of the mixing process while stirring at 9,500 rpm and this speed was maintained for 5 minutes in total. Mixing was carried out with a high speed homogeniser.
   Then the mixture obtained was subjected to a treatment at 700 bar for 30 seconds in a high pressure homogenizer (Microfluidizer®, trade name of Microfluidics Corp., USA).

### Evaluation of the stability of the emulsions.

The emulsions obtained were divided in two parts, one of which was stored at room temperature (RT) and the other one at 50°C.
The stability of the emulsions was evaluated macroscopically by visual inspection for oil droplets and oil separation.

### Specific emulsions evaluated.

The nature of the hydrophobically modified saccharides of formula (I) and (II) used as surfactant in Example 1 is indicated in Table 1 below.
The particulars of the emulsions tested and the results obtained in Example 1 are shown in Table 2 below.

**Table 1:**

| Hydrophobically modified saccharides of formula (I) and (II) | | | | | | |
|---|---|---|---|---|---|---|
| SURFACTANT | | | | | | |
| Product n° | Lab ref. | Formula (I) or (II) | Type | Hydrophobic Moiety | | av. DS |
| | | | | M | R- | |
| 1 | MP 79 | (I) | a | R-NH-CO | CH₃(CH₂)₇- | 0.02 |
| 2 | AM 150 | (I) | a | R-NH-CO | CH₃(CH₂)₇- | 0.08 |
| 3 | AM 149 | (I) | a | R-NH-CO | CH₃(CH₂)₇- | 0.09 |
| 4 | AM 154 | (I) | a | R-NH-CO | CH₃(CH₂)₇- | 0.2 |
| 5 | AM 238 | (I) | a | R-NH-CO | CH₃(CH₂)₁₁- | 0.07 |
| 6 | AM 219 | (I) | a | R-NH-CO | CH₃(CH₂)₁₁- | 0.09 |
| 7 | AM 259 | (I) | a | R-NH-CO | CH₃(CH₂)₁₁- | 0.1 |
| 8 | MP 28 | (I) | a | R-NH-CO | CH₃(CH₂)₁₁- | 0.1 |
| 9 | MP 73 | (I) | a | R-NH-CO | CH₃(CH₂)₁₁- | 0.1 |
| 10 | MP 66b | (I) | a | R-NH-CO | CH₃(CH₂)₁₁- | 0.12 |
| 11 | AM 220b | (I) | a | R-NH-CO | CH₃(CH₂)₁₁- | 0.15 |
| 12 | AM 82 | (I) | a | R-NH-CO | CH₃(CH₂)₁₁- | 0.21 |
| 13 | MP 20 | (I) | a | R-NH-CO | CH₃(CH₂)₁₁- | 0.3 |
| 14 | MP 32 | (I) | a | R-NH-CO | CH₃(CH₂)₁₅- | 0.21 |
| 15 | MP 78 | (I) | a | R-NH-CO | CH₃(CH₂)₁₇- | 0.023 |
| 16 | AM 22 | (I) | a | R-NH-CO | CH₃(CH₂)₁₇- | 0.054 |
| 17 | MP 80 | (I) | a | R-NH-CO | CH₃(CH₂)₁₇- | 0.11 |
| 18 | AM 244 | (I) | b | R-NH-CO | CH₃(CH₂)₁₁- | 0.3 |
| 19 | MP 36 | (I) | a | R-CO | CH₃(CH₂)₁₀- | 0.12 |
| 20 | MP 41 | (I) | a | R-CO | CH₃(CH₂)₁₄- | 0.1 |
| 21 | MP 40 | (I) | a | R-CO | CH₃(CH₂)₇CH=C H-(CH₂)₇- | 0.05 |
| 22 | MP 42 | (I) | a | R-CO | CH₃(CH₂)₁₆- | 0.11 |
| 23 | AM 141 | (II) | d | R-NH-CO | CH₃(CH₂)₁₁- | 0.05 |
| 24 | AM 117 | (II) | e | R-NH-CO | CH₃(CH₂)₁₁- | 0.1 |
| 25 | PC 17 | (II) | c | R-NH-CO | CH₃(CH₂)₁₁- | 0.1 |
| 26 | PC 16 | (II) | d | R-NH-CO | CH₃CH₂)₁₁- | 0.18 |
| 27 | MP 98 | (II) | d | R-CO | CH₃(CH₂)₁₀- | 0.1 |
| 28 | AM 70 | (I) | a | R-NH-CO | CH₃(CH₂)₇- | 0.11 |
| 29 | MP 31 | (I) | a | R-NH-CO | CH₃(CH₂)₁₅- | 0.12 |
| 30 | MP 92B | (I) | f | R-NH-CO | CH₃(CH₂)₁₁- | 0.19 |
| 31 | MP 102 | (I) | f | R-NH-CO | CH₃(CH₂)₁₁- | 0.13 |
| Formula [A] ₙ (-M) ₛ (I) [B] ₘ (-M) _{s'} (II) | | | | | | |
| a = inulin, av. DP : 23 (RAFTILINE® HP, ORAFTI, Belgium) | | | | | | |
| b = inulin, DP mainly between 2 and 8, av. DP : about 4.5 (RAFTILOSE® P95, ORAFTI, Belgium) | | | | | | |
| c = maltodextrin, DE 2 (Roquette, France) | | | | | | |
| d = maltodextrin, DE 28 (Roquette, France) | | | | | | |
| e = maltodextrin, DE 47 (Roquette, France) | | | | | | |
| f = inulin, av. DP : 13 (RAFTILINE® ST, ORAFTI, Belgium) | | | | | | |

**Table 2 :**

| Emulsions: Particulars and Stability | | | | | | | | |
|---|---|---|---|---|---|---|---|---|
| Surfactant | | Ratio composing phases oil phase/ aqueous phase | | Dispersion | Salt | | Stability (in months) | |
| Prod. n° (*) | %w/v on dispersed liquid phase | Ratio (v/v) (**) | Kind of oil | Method of prep. | Kind | Molarity in aqueous phase | at Room Temp | at 50°C |
| | | | | | | | | |
| 1 | 2 | 50:50 | Isoparaffinic oil¹ | A | NaCl | 1 | > 4 | > 4 |
| 2 | 2 | 50:50 | Isoparaffinic oil¹ | A | NaCl | 1 | > 5 | > 5 |
| 2 | 2 | 50:50 | Isoparaffinic oil¹ | A | MgSO₄ | 1 | >5 | > 5 |
| 3 | 2 | 50:50 | Isoparaffinic oil¹ | A | NaCl | 1 | > 5 | > 5 |
| 3 | 2 | 50:50 | Isoparaffinic oil¹ | A | MgSO₄ | 1,5 | | > 2.5 |
| 3 | 2 | 50:50 | Isoparaffinic oil¹ | A | MgSO₄ | 2 | | 2,5 |
| 4 | 8 | 50:50 | 85% Isoparaffinic oil¹+ 15% Squalane oil² | D | NaCl | 1 | > 2 | > 2 |
| 4 | 8 | 50:50 | 85% Isoparaffinic oil¹ + 15% Squalane oil² | D | MgSO₄ MgSO₄ | 1 | 1,5 | 1.5 |
| 5 | 2 | 50:50 | Isoparaffinic oil¹ | B | NaCl | 1 | > 14 | > 1 |
| 6 | 2 | 50:50 | Isohexadecane oil³ | A | NaCl | 1 | > 14 | > 1 |
| 7 | 2 | 50:50 | Oilmix⁴ | A | NaCl | 1 | > 12 | > 1.3 |
| 7 | 2 | 50:50 | Isoparaffinic oil¹ | A | CaCl₂ | 1 | >12 | > 4 |
| 7 | 2 | 50:50 | Isoparaffinic oil¹ | | CaCl₂ | 2 | >12 | 4 |
| 8 | 0.25 | 50:50 | Isoparaffinic oil¹ | A | ^{NaCl} | 1 | > 7 | > 7 |
| 8 | 1.6 | 50:50 | Isoparaffinic oil¹ + 0.4% Sorbitan monolaurate⁵ | A | NaCl | 1 | >7 | > 7 |
| 8 | 0.8 | 50:50 | Isoparaffinic oil¹ + 0.2% Sorbitan monolaurate⁵ | A | ^{NaCl *} | ¹ | ^{> 7} | > 7 |
| 8 | 0.4 | 50:50 | Isoparaffinic oil¹ + 0.1% Sorbitan monolaurate⁵ | A | NaCl | 1 | > 7 | > 7 |
| 8 | 2 | 50:50 | Isoparaffinic oil¹ | A | MgCl₂ | 5 | > 2 | > 0.7 |
| 9 | 2 | 50:50 | Isoparaffinic oil¹ | A | Ca- lactaat | 1 | > 3 | > 3 |
| 9 | 2 | 50:50 | Isoparaffinic oil¹ | A | Na- lactaat | 1 | > 3 | > 3 |
| 9 | 2 | 50:50 | Isoparaffinic oil¹ | A | Ammonium sulfate | 1 | > 3 | > 3 |
| 10 | 2 | 50:50 | Cyclomethicone oil⁶ | A | NaCl | 1 | > 6 | > 6 |
| 10 | 2 | 50:50 | Isoparaffinic oil ¹ | A | MgCl₂ | 1 | > 3 | > 3 |
| 10 | 2 | 50:50 | Isopropyl myristate oil⁷ | A | NaCl | 1 | > 4 | > 3 |
| 10 | 2 | 50:50 | Isoparaffinic oil¹ | A | NaCl | 5 | > 5 | > 5 |
| 10 | 2 | 20:80 | Isoparaffinic oil¹ | A | MgSO₄ | 1 | > 2 | > 2 |
| 10 | 5 | 20:80 | Isoparaffinic oil¹ | A | MgSO₄ | 1 | > 2 | > 2 |
| 10 | 2 | 80:20 | Isoparaffinic oil¹ | A | MgSO₄ | 1 | > 2 | > 2 |
| 10 | 1.25 | 80:20 | Isoparaffinic oil¹ | A | MgSO₄ | 1 | > 2 | > 2 |
| 10 | 2 | 50:50 | Isoparaffinic oil¹ | A | NH₄Cl | 1 | > 1.5 | > 1.5 |
| 10 | 0.5 | 50:50 | Isoparaffinic oil¹ | A | NH₄Cl | 1 | > 1.5 | > 1 |
| 10 | 2 | 50:50 | Isoparaffinic oil¹ | A | Et3N. HCl.aq | 1 | > 1 | > 1 |
| 10 | 2 | 50:50 | Isoparaffinic oil¹ | A | Na citrate | 0.5 | > 1 | > 1 |
| 10 | 2 | 50:50 | Isoparaffinic oil¹ | A | Glypho sate ⁸ | 0.7 | > 1.5 | > 1.5 |
| 11 | 2 | 50:50 | Isoparaffinic oil¹ | A | NaCl | 1.5 | > 16 | > 2.5 |
| 11 | 2 | 50:50 | Isoparaffinic oil¹ | A | NaCl | 2 | > 16 | 2.5 |
| 11 | 2 | 50:50 | Isoparaffinic oil¹ | A | MgSO₄ | 1.5 | > 16 | 2.5 |
| 11 | 2 | 50:50 | Isoparaffinic oil¹ | A | MgSO₄ | 2 | ^{>16} | ^{2.5} |
| 12 | 8 | 50:50 | 85 % Isoparaffinic oil¹ + 15% Squalane oil² | C | NaCl | 1 | >2 | > 2 |
| 12 | 8 | 50:50 | 85 % Isoparaffinic oil¹ + 15% Squalane oil² | C | MaSO₄ | 1 | >2 | > 2 |
| 13 | 2 | 50:50 | Isoparaffinic oil¹ | A | NaCl | 1 | > 5 | > 5 |
| 13 | 2 | 50:50 | Isoparaffinic oil¹ | A | MgSO₄ | ¹ | ^{> 5} | ^{> 5} |
| 14 | 2 | 50:50 | Isoparaffinic oil¹ | A | NaCl | 1 | > 5 | > 5 |
| 14 | 2 | 50:50 | Isoparaffinic oil¹ | A | MgSO4 | 1 | > 5 | > 5 |
| 15 | 2 | 50:50 | Isoparaffinic oil¹ | A | NaCl | 1 | > 5 | > 5 |
| 16 | 2 | 50:50 | High oleic sunflower seed oil⁹ | A | NaCl | 1 | > 2 | > 2 |
| 16 | 2 | 50:50 | High oleic sunflower seed oil | A | MgSO₄ MgSO₄ | 1 | > 2 | > 2 |
| 17 | 2 | 50:50 | Isoparaffinic oil¹ | A | NaCl | 1 | > 5 | > 5 |
| 18 | 2 | 50:50 | Isoparaffinic oil¹ | A | NaCl | 1 | > 14 | > 1 |
| 20 | 2 | 50:50 | Isoparaffinic oil¹ | A | NaCl | 1 | > 8.5 | > 6 |
| 21 | 2 | 50:50 | Isoparaffinic oil¹ | A | NaCl | 1 | > 8.5 | > 8.5 |
| 22 | 2 | 50:50 | Isoparaffinic oil¹ | A | NaCl | 1 | > 8.5 | > 8.5 |
| 23 | 2 | 50:50 | Isoparaffinic oil¹ | A | MgSO₄ | 1 | > 1.5 | > 1.5 |
| 24 | 2 | 50:50 | Isoparaffinic oil¹ | A | MgSO₄ | 1 | > 1.5 | > 1.5 |
| 25 | 2 | 50:50 | Isoparaffinic oil¹ | A | NaCl | 1 | > 4 | > 4 |
| 26 | 2 | 50:50 | Isoparaffinic oil¹ | A | NaCl | 1 | > 4 | > 4 |
| 27 | 2 | 50:50 | Isoparaffinic oil¹ | A | NaCl | 1 | > 5 | > 5 |
| 27 | 2 | 50:50 | Isoparaffinic oil¹ | A | MgSO₄ | 1 | > 5 | > 2 |
| 30 | 2 | 50:50 | Isoparaffinic oil¹ | A | NaCl | 1 | > 4 | > 4 |
| 30 | 2 | 50:50 | Isoparaffinic oil¹ | A | MgSO₄ | 1 | > 4 | > 4 |
| 31 | 2 | 50:50 | Isoparaffinic oil¹ | A | NaCl | 1 | > 3 | > 3 |

| | | | | | | | | |
|---|---|---|---|---|---|---|---|---|
| Legend (*)The product number corresponds to the product number given in Table 1. | | | | | | | | |
| (**) volume ratio of the composing phases before homogenising. Methods A, B, C and D for preparing the dispersions are as defined above. | | | | | | | | |
| ¹ Isopar M - Exxon Chemicals | | | | | | | | |
| ² Pripure3759 Squalane (vegetable oil) - Uniqema | | | | | | | | |
| ³ Arlamol HD - Uniqema | | | | | | | | |
| ⁴ oilmix = (2/10/4/2) - high oleic sunflower seed oil - FLORASUN 90 (International Flora Technologies Ltd.)/ isohexadecane oil - Arlamol HD (Uniqema)/glycerol tricaprylate, caprate - Estol 3603 (Uniqema)/Avocado oil (Alpha pharma) | | | | | | | | |
| ⁵ Span 20 - Uniqema | | | | | | | | |
| ⁶ EU 344 cyclomethicone oil - Dow Coming | | | | | | | | |
| ⁷ Estol 1514 - Uniqema | | | | | | | | |
| ⁸ Glyphosate was not used as such, but the commercial product Round Up plus® (Monsanto) was used as the aqueous phase. | | | | | | | | |
| ⁹ FLORASUN 90 - International Flora Technologies Ltd | | | | | | | | |

### Example 2

### Comparative tests

The efficiency as surfactant of the hydrophobically modified saccharides of formula (I) and (II) was compared to those of commercial surfactants.
The commercial products used in the comparative tests of Example 2 are indicated in Table 3 below.
The same procedures, methods and conditions were used as the ones described in Example 1 above. The data of the tests of Example 2 are shown in Table 4 below and these data are to be compared with the data obtained in Example 1 and presented in Table 2.

**Table 3 :**

| Commercial products used in the comparative examples of Example 2. | | | |
|---|---|---|---|
| Product reference | Product name (trade name) | Nature | Producer |
| Ref 1 | DUB SE 15P | Saccharose monopalmitate | Stearinerie Dubois, France |
| Ref 2 | DUB SE 16S | Saccharose monostearate | Stearinerie Dubois, France |
| Ref 3 | Pluronic PE 6400 | Block copolymer | BASF, Germany |
| Ref 4 | Pluronic PE 6800 | Block copolymer | BASF, Germany |
| Ref 5 | Plantacare 1200UP | Lauryl glucoside | Fluka |
| Ref 6 | Pemulen TR1 | Polymeric emulsifier | BF Goodrich, Ohio USA |
| Ref 7 | Arlatone Versaflex V-175 | Nonionic, polymeric -based emulsifying system¹ | Uniqema, UK |

| | | | |
|---|---|---|---|
| ¹ Arlatone Versaflex V-175 is a blend of polysaccharides and esters. | | | |

**Table 4 :**

| Stability of comparative emulsions | | | | | | | | |
|---|---|---|---|---|---|---|---|---|
| Surfactant | | Ratio of phases oil / aqueous phase | | Dispersion | Salt | | Stability | |
| Prod. ref (*) | % w/v on dispersed liquid phase | Ratio (v/v) (**) | Kind of oil | Method of prep. | Kind | Molarity in aqueous phase | at room temp. | at 50°C |
| Ref 1 | 2 | 50/50 | Isoparaffinic oil² | A | NaCl | 1 | > 5 months | 1 day |
| Ref 2 | 2 | 50/50 | Isoparaffinic oil² | A | NaCl | 1 | > 5 months | 1 day |
| Ref 3 | 2 | 50/50 | Isoparaffinic oil² | A | NaCl | 1 | > 5 months | <5 months |
| Ref 4 | 2 | 50/50 | Isoparaffinic oil² | A | NaCl | 1 | > 5 months | <5 months |
| Ref 3³ | 2 | 50/50 | Isoparaffinic oil² | A | MgSO₄ | 1 | <5 months | <1 months |
| Ref 4³ | 2 | 50/50 | Isoparaffinic oil² | A | MgSO₄ | 1 | > 5 months | <1 months |
| Ref 5 | 2 | 50/50 | Isoparaffinic oil² | A | NaCl | 1 | >3 months | >3 months |
| Ref 5 | 2 | 50/50 | Isoparaffinic oil² | A | MgSO₄ | 1 | >3 months | >3 months |
| Ref 5 | 2 | 50/50 | Isoparaffinic oil² | A | NaCl | 5 | <8days | <8days |
| Ref 5 | 1 | 50/50 | lsoparaffinic oil² | A | MgSO₄ | 2 | > 1 day | 1 day |
| Ref 5 | 2 | 50/50 | Isoparaffinic oil² | A | MgSO₄ | 2 | <8days | <8days |
| Ref 6 | 4 | 20/80 | Isoparaffinic oil² | E⁴ | MgSO₄ | 1 | impossible | impossible |
| Ref 7 | 5 | 20/80 | Isoparaffinic oil² | F⁵ | MgSO₄ | 0.5 | > 1 week | < 1 week |

| | | | | | | | | |
|---|---|---|---|---|---|---|---|---|
| (*) The product reference corresponds to the one indicated in Table 3. | | | | | | | | |
| (**) volume ratio of the composing phases before homogenising. Method A for preparing the dispersions was as defined above | | | | | | | | |
| ¹ Arlatone V-175 is a blend of polysaccharides and esters. ² IsoparM - Exxon Chemicals | | | | | | | | |
| ³ Emulsions were very flocculated and had an unsmooth, not bright white look | | | | | | | | |
| ⁴ preparation of emulsion according to method E: First the surfactant was added to the aqueous phase (containing the salt) under continuous stirring (propeller stirrer) at 850 rpm, then the oil phase was added dropwise while still stirring at 850 rpm during 10 minutes. Addition of the salt after emulsifying yielded no good result neither (emulsion collapsed) | | | | | | | | |
| ⁵ preparation of emulsion according to method F (method suggested by Uniqema (ICI, UK) for the preparation of an emulsion with Arlaton Versaflex at labscale (200 g) according to the cold procedure): ■ Put gently bit by bit Arlatone Versaflex in the water phase under stirring (800-1000 rpm) ■ Continue to stir for about 10 minutes (800-1000 rpm) ■ Add all water-soluble ingredients to the water phase under stirring (800-1000 rpm) ■ Add the oil phase to the water phase under stirring (800-1000 rpm) ■ Homogenise for 2 minutes at high speed (about 10,000 rpm) ■ Stir (800-1000 rpm) until appearance is homogeneous. | | | | | | | | |

### Example 3

Suspensions in accordance with the present invention were made and evaluated as follows.
2.5 g Carbon Black (Elftex 570, Cabot corporation) was added slowly to 40 ml of a 1.25 % (% in w/v) aqueous surfactant solution (surfactant product 9 of Table 1) (containing either 0 or 1 Mole of NaCl) while stirring the solution at 8500 rpm by means of a high speed homogeniser. After addition of the powder, the dispersion was stirred for 3 extra minutes at 9500 rpm.
Microscopic evaluation of the suspensions made with and without NaCl in the aqueous phase showed that the addition of the surfactant highly reduced flocculation of the particles for at least 5 days at room temperature. In comparative tests, the suspensions made in the absence of the surfactant showed considerable flocculation.

### Example 4

A suspension consisting of polystyrene particles dispersed in aqueous medium was prepared using a surfactant-free method (A. Kotera, *et al.,* Kolloid ZZ.
Polym., 227 (1968) 759) by mixing milli-Q water, styrene-monomer (10 % v/v) and potassium persulfate (K₂S₂O₈; 0.06 % w/w on total) under nitrogen atmosphere (about 1 bar) at 70°C during 24 hours. In this way negatively charged polystyrene particles with a mean diameter of 210 nm were obtained.
The stability of the obtained polystyrene dispersion in the presence of a salt (so-called salt-stability) with and without addition of a surfactant according to the invention was investigated and the critical coagulation concentration (CCC) was determined. The test was carried out by mixing the surfactant-free polystyrene dispersion, diluted with water to a dispersion at 5 % w/w polystyrene, with a given amount of surfactant and electrolyte (NaCl or CaCl2), at room temperature and keeping the samples in a water bath at 25°C for 12 hours. Coagulation of the particles was assessed through visual observation and by optical microscopy. The CCC, being the lowest salt concentration in mole/l at which coagulation was observed, was determined. Table 5 shows the CCC-results for the stabilisation of aqueous dispersions at 5 % w/w polystyrene with various surfactants and salts.

**Table 5:**

| CCC results of aqueous dispersions at 5 % w/w polystyrene | | | | |
|---|---|---|---|---|
| Surfactant | Conc. of surfactant (% w/w on total) | Conc. of surfactant (% w/w on dispersed phase) | CCC (in mole/l) | |
| | | | NaCl | CaCl₂ |
| Without surfactant* | - | - | 0.375 | 0.0075 |
| Product n° 9 of Table 1 above | 0.25 | 5.0 | > 5.17 | > 4.37 |
| Product n° 9 of Table 1 above | 0.01 | 0.2 | nd | > 1.6 |
| Brij®30 (1)* | 0.25 | 5.0 | 0.32 | 0.25 |
| Na-dodecylsulfate (2)* | 0.25 | 5.0 | nd | 0.035 |
| Synperonic PE L64 (3)* | 0.25 | 5.0 | nd | 0.088 |
| Plantacare 1200UP (4)* | 0.25 | 5.0 | nd | 0.05 |
| Plantacare 2000 (5)* | 0.25 | 5.0 | nd | 0.065 |

| | | | | |
|---|---|---|---|---|
| Legend: * : comparative test | | | | |
| nd : not determined | | | | |
| (1) : Brij®30 (=fatty alcohol-ethoxylate) (trade name, ICI, UK) | | | | |
| (2) : Sodiumdodecylsulfate (=anionic surface active agent) - 99% pure (Across Organics) | | | | |
| (3): Synperonic PE L64 (=EO/PO block copolymer) (trade name, ICI, UK) | | | | |
| (4): Plantacare 1200UP (=laurylglucoside) (trade name,Fluka) | | | | |
| (5): Plantacare 2000 (=decylglucoside) (tradename, Fluka) | | | | |

The results of the experiments described in Table 5 regarding a typical, hydrophobically modified saccharide clearly show that, according to the present invention, the hydrophobically modified saccharides are suitable as surfactants for the stabilisation of dispersions that contain a high concentration of an electrolyte. The CCC-value obtained indicates that dispersion stability is guaranteed even at a very low concentration of the hydrophobically modified saccharide.

### Example 5

Aqueous poly(methylmethacrylate) (PMMA)-dispersions were made by mixing methyl methacrylate (MMA) (5 % w/w), water (94.7% w/w), potassium persulfate (K₂S₂O₈) (0.025 % w/w) and sodium dodecylsulfate (SDS) (0.286 % w/w). The polymerisation reaction was carried out under nitrogen atmosphere at about 1 bar at 70°C under stirring during 24 hours. A dispersion of PMMA-particles with a mean diameter of 61.8 nm was obtained. The dispersion obtained was diluted with water to a suspension at 2.5% w/w PMMA-particles, which was used for the determination of the critical coagulation concentration (CCC). By gradual addition of CaCl₂ to the suspension, a CCC value was found for CaCl₂ of 0.0075 mole/l. Addition to the suspension of 0.5 % w/w (20 % w/w on dispersed phase) of an hydrophobically modified saccharide (product 9 of Table 1 above) resulted in a CCC of more than 2.29 mole/l. This illustrates, according to the invention, the dispersion-stabilising effect of the hydrophobically modified saccharides on suspensions containing a high concentration of an electrolyte.

### Example 6

The influence of salt on foam stability (a liquid/gas two-phase system) was investigated using a Foamtester R2000 (Sita Messtechnik GmbH, Germany). The apparatus generates in a standardised way foam in a 1500 ml recipient and follows the foam stability as a function of time. In a series of experiments, in accordance with the present invention, a given concentration of a hydrophobically modified saccharide was dissolved in an aqueous 1 mole/l NaCl solution and 300 ml of the solution was put into the Foamtester. The apparatus generated foam by stirring the mixture in contact with air at 2,000 rpm during one minute. Accordingly, the generated foam volume (V₀) was automatically determined and foam stability was followed as a function of time by measurement of the remaining foam volume (expressed as % of V₀). The results are shown in Table 6 below.

**Table 6 :**

| Foam stability of a system containing a hydrophobically modified saccharide in an aqueous 1 mole/l NaCl solution | | | |
|---|---|---|---|
| Surfactant | Concentration of surfactant (% w/v) | Generated foam volume V₀** (ml) | % of V₀ after 80 minutes |
| SDS * | 0.3% | 300 | 50 |
| Product n°9 of table 1 | 0.1% | 400 | 75 |
| Product n° 3 of table 1 | 0.1% | 580 | 90 |

| | | | |
|---|---|---|---|
| * : comparative example | | | |
| ** : V₀ = volume of generated foam at time zero (= just after foam generation) | | | |

From table 6 it follows that, compared to the use of SDS, the use according to the present invention of hydrophobically modified saccharides generate in the presence of a high concentration of an electrolyte salt, a higher volume of foam and give more stability to the foam.

### Examples 7 and 8

Example 7 shows a cosmetic composition according to the present invention, being a highly stable anti-perspirant emulsion containing a high amount of an aluminium salt (as anti-perspirant agent) in the water phase and a high load of an oil phase (as emollient), in the presence of a hydrophobically modified saccharide as surfactant.
Example 8 (comparative) shows a same composition as in example 7, but with a same amount of a commercial surfactant.
To prepare the emulsions of Examples 7 and 8, the composition of which is indicated below, phases A and B were prepared separately at room temperature (RT) by homogeneously mixing of the ingredients. Accordingly, at RT, Phase B was added to phase A in 2 minutes while mixing at 3,000 rpm and the mixture was additionally homogenised by stirring at 15,000 rpm during 3 minutes.
Comparison of the compositions of Examples 7 and 8 showed that the formulation of Example 7 was still stable towards coalescence after storage for 120 hours at 45°C, while the emulsion of Example 8, stored under the same conditions, showed significant oil separation.

| Example 7 | | Example 8* | |
|---|---|---|---|
| Ingredients | % | Ingredients | % |
| *Phase A* | | *Phase A* | |
| Water | 22 | Water | 22 |
| Aluminium Chloro-Hydrate (50 wt%) | 50 | Aluminium Chloro-Hydrate (50 wt%) | 50 |
| Product n°9-table 1 | 1 | Arlacel 165 (1) | 1 |
| *Phase B* | | *Phase B* | |
| Caprylic capric Triglyceride | 12.5 | Caprylic capric Triglyceride | 12.5 |
| Isostearyl iso-Stearate | 12.5 | Isostearyl iso-Stearate | 12.5 |
| Phenoxy ethanol + Paraben (2) | 0.5 | Phenoxy ethanol + Paraben (2) | 0.5 |
| Fragrance | 0.4 | Fragrance | 0.4 |

| | | | |
|---|---|---|---|
| *: comparative example | | | |
| (1): Arlacel 165 (trade name, ICI, UK)( =glyceryl stearate and PEG-100 stearate) | | | |
| (2) : Paraben ( trade name, Bufa, Belgium) (= 4-hydroxybenzoic acid) | | | |

### Examples 9 and 10

Capillary treatment products often contain high amounts of electrolytes as active materials. The quality of capillary treatment products can be improved by the addition of emollients. Example 9 presents an example of a capillary treatment product in the form of an emulsion, containing a hydrophobically modified saccharide as surfactant in accordance with the present invention, that is enriched by a significant amount of an oil phase. Example 10 (comparative) presents a same emulsion as Example 9 but in which the hydrophobically modified saccharide was replaced by the surfactant sorbitan isostearate.
The composition of Examples 9 and 10 is indicated below. To prepare the emulsions of Examples 9 and 10, phases A and C were prepared separately at room temperature by homogeneously mixing of the ingredients. The ingredients of phase B were then added to phase A, and then phase C was added under stirring at 3,000 rpm to said mixture of phases A and B, yielding the emulsion of respectively Example 9 and Example 10. After storage for 48 hours at 50°C, the formulation according to Example 10 showed significant oil separation (coalescence), whereas Example 9 showed no oil separation.

| Example 9 | | Example 10* | |
|---|---|---|---|
| Ingredients | % | Ingredients | % |
| *Phase A* | | *Phase A* | |
| Water | 47 | Water | 47 |
| Na₂EDTA | 0.1 | Na₂EDTA | 0.1 |
| NH₄ Thioglycolate | 17 | NH₄ Thyoglycolate | 17 |
| NH₄ Bicarbonate | 4.5 | NH₄ Bicarbonate | 4.5 |
| Styrene/vinyl pyrrolidone copolymer | 0.3 | Styrene/vinyl pyrrolidone copolymer | 0.3 |
| Ammonia | 0.5 | Ammonia | 0.5 |
| pH adjustment till pH 8.8 | | pH adjustment till pH 8.8 | |
| PEG-15 Coco Polyamine | 3.6 | PEG-15 Coco Polyamine | 3.6 |
| Product n°9 (table 1) | 0.5 surfactant | ― | |
| *Phase B* | | *Phase B* | |
| Polysorbate 20 | 0.6 | Polysorbate 20 | 0.6 |
| Fragrance | 0.4 | Fragrance | 0.4 |
| *Phase C* | | *Phase C* | |
| Isostearyl Isostearate | 12.5 | Isostearyl Isostearate | 12.5 |
| Ethoxy diglycol Oleate | 12.5 | Ethoxy diglycol Oleate | 12.5 |
| Sorbitan isostearate | 0.5 surfactant | Sorbitan isostearate | 1.0 surfactant |

| | | | |
|---|---|---|---|
| *: comparative | | | |

### Examples 11 and 12

Using hydrophobically modified saccharides according to the invention, a facial or hand cream containing high amounts of a moisturization agent (typically sodium pyrrolydone carboxylate (Nalidon®, trade name of UCB, Belgium) and presenting excellent stability can be prepared as shown by Examples 11 and 12.
The samples are obtained by preparing separately phases A under gently warming up (warm process), B (cold process at RT) and C (cold process at RT).
Then Phase B is added to Phase A in 2 minutes while mixing at 3,000 rpm, with additional homogenizing during 5 minutes at 15,000 rpm. Then Phase C is added to the obtained mixture under slow stirring, yielding the emulsions of Examples 11 and 12.
Example 11 presents a composition according to the invention of a cream containing a hydrophobically modified saccharide, which shows after storage of 120 hours at 45°C and after 15 minutes of centrifugation at 13,000 rpm, no coalescence. Comparative Example 12 tested under the same conditions showed strong coalescence with eventual formation of an oil layer and an aqueous layer. In Examples11 and 12 the thickener is sodium magnesium silicate because it is stable towards electrolytes. Conventional thickeners based on polycarboxylic acids and hydrophobically poly-carboxylic acids loose their thickener behaviour under the applied conditions.

| Example 11 | | Example 12 * | |
|---|---|---|---|
| Ingredients | % | Ingredients | % |
| *PhaseA (warm process)* | | *Phase A (warm process)* | |
| Water | 59 | Water | 59 |
| Sodium Magnesium silicate | 3.0 | Sodium Magnesium silicate | 3.0 |
| Product n°9-table 1 | 0.5 | Sorbitan isostearate | 0.5 |
| Phenoxy ethanol + Paraben ** | 0.5 | Phenoxy ethanol + Paraben ** | 0.5 |
| *Phase B (cold process)* | | *Phase B (cold process)* | |
| Isostearyl Isostearate | 12.5 | Isostearyl Isostearate | 12.5 |
| Caprilic capric triglyceride | 12.5 | Caprilic capric triglyceride | 12.5 |
| Fragrance | 0.4 | Fragrance | 0.4 |
| *Phase C* | | *Phase* C | |
| Sodium Pyrrolydone Carboxylate | 12 | Sodium Pyrrolydone Carboxylate | 12 |

| | | | |
|---|---|---|---|
| * : comparative | | | |
| ** : Paraben ( trade name, Bufa, Belgium) (= 4-hydroxybenzoic acid) | | | |

The results of the Examples above clearly show that the hydrophobically modified saccharides of formula (I) and (II) present tensio active properties which make these compounds useful as surfactants for the preparation of dispersions comprising an aqueous phase containing a high concentration of electrolytes, that are stable at room temperature or show improved stability compared to dispersions prepared with known surfactants. The dispersions according to the present invention even present excellent stability at elevated temperatures such as at 50 °C and even at higher temperatures (*e.g.* dispersion with product 5 in Table 2 remains stable for at least 1 month at 65° C).

The dispersions in which hydrophobically modified saccharides of formula (I) and/or (II) are used as surfactants in accordance with the present invention may optionally further comprise one or more conventional surfactants, co-surfactants and/or additives such as for example thickeners and rheology modifiers.

The hydrophobically modified saccharides of formula (I) and/or (II) are suitable as surfactants for the preparation of any kind of dispersions comprising a continuous aqueous phase, typically for the preparation of dispersions in the field of cosmetics and health care, of food preparations, cutting oils, paintings, inks, crop protection, pesticides, insecticides and herbicides.

Since many cosmetic compositions are based on emulsion systems, there is a great interest to formulate electrolyte-active materials into cosmetic emulsions. Examples 7, 9 and 11 indicate hydrophobically modified saccharides of formula (I) and/or (II) are suitable as surfactant for the preparation of such emulsion systems containing a high concentration of an electrolyte in the aqueous phase. Examples of cosmetic compositions of the emulsion type wherein, in accordance with the present invention, hydrophobically modified saccharides are suitable as surfactant are, for example, creams, deodorants, antiperspirants, capillary treatment products, shampoos, health and personal care products containing electrolyte type moisturizing agents, and hair products containing cationic and/or amphoteric active materials.

Based on the tensio-active properties of the compounds of formula (I) and (II) defined above, the present invention also provides a method for the preparation of dispersions and/or for stabilising dispersions comprising an aqueous phase containing a high concentration of electrolytes, by including a said compound or mixture of said compounds of formula (I) and/or (II) in the composition of the dispersion. The particular conditions, such as regarding the concentration of said surfactant(s), the ratio non-aqueous phase(s) / continuous aqueous phase, and others, can be derived from the information provided above.

The dispersions can be prepared by conventional methods and techniques. The dispersions can for example be prepared by bringing together and homogenising the composing phases of the multiphase system, with addition of one or more hydrophobically modified saccharides of general formula (I) and/or (II) defined above to the aqueous phase, to the non-aqueous phase(s) and/or to the composing phases of the multiphase system, so as to bring the non-continuous phase(s) in the form of finely divided particles (droplets, solid particles and/ gas bubbles) dispersed in the continuous aqueous phase. The surfactant(s) of general formula (I) and/or (II) are typically added to the aqueous phase before the composing phases are mixed and homogenised to yield the dispersion.

A considerable advantage of the hydrophobically modified saccharides of formula (I) and/or (II) is that they are very versatile compounds which can be engineered in view of particular dispersions and their application. This versatility results from the several parameters which define the structure of the molecule, namely the saccharide type and its degree of polymerisation, the kind of hydrophobic moiety and the average degree of substitution. Further advantages of the hydrophobically modified saccharides of formula (I) and/or (II) reside in the fact that they are derived from saccharides from renewable resources, and that the products generally present good biodegradability and low toxicity, if any at all, towards humans, mammals, birds and fish.

## Claims

1. Use of a surfactant for the preparation of a dispersion of a multiphase system that comprises a continuous aqueous phase, **characterised in that** said aqueous phase contains one or more electrolytes at a total concentration ranging from the lower limit of 0.1 to 1 mole per litre aqueous phase, depending on the nature of the electrolyte(s) and the temperature of the dispersion, up to the limit of the solubility of the electrolyte(s) in water at 25°C, and that said surfactant is a hydrophobically modified saccharide which is a substituted polymeric saccharide of general formula (I) or (II)
[A]ₙ (-M)ₛ (I) [B]ₘ (-M)_{s'} (II)
wherein
[A] ₙ represents a fructan-type saccharide with [A] representing a fructosyl unit or a terminal glucosyl unit and n representing the degree of polymerisation (DP) being the number of the fructosyl and glucosyl units in said fructan-saccharide molecule,
[B] ₘ represents a starch-type saccharide, with [B] representing a glucosyl unit and m being the number of glucosyl units in said starch-type saccharide molecule, selected from the group consisting of modified starches and of starch hydrolysates with a dextrose equivalent (DE) ranging from 2 to 47,
(-M) represents a hydrophobic moiety that substitutes a hydrogen atom of a hydroxyl group of said fructosyl or glucosyl units, which is selected from the group consisting of an alkylcarbamoyl radical of formula R-NH-CO- and an alkylcarbonyl radical of formula R-CO- , wherein R represents a linear or branched, saturated or alkyl group with from 4 to 32 carbon atoms,
s and s' , which can have the same value or not, represent the number of said hydrophobic moieties that substitute the fructosyl or glucosyl unit, expressed as the average degree of substitution (av. DS) which ranges from 0.01 to 0.5,
and
the total concentration of substituted polymeric saccharide of formula (I) and/or formula (II) ranges from 0.10 to 20 %, being % w/v on dispersed phase(s) in case of emulsions, % w/w on dispersed phase(s) in case of suspensions, and % w/v on aqueous phase in case of foams.

2. Use according to claim 1, wherein the multiphase system is selected from the group consisting of a biphase system, and a triphase system, and the ratio non-aqueous phase(s) / aqueous phase ranges from 90: 10 to 1: 99, expressed as volume: volume ratio in case the non-aqueous phase(s) are liquid or gas phases, and as weight : volume ratio in case the non-aqueous phase(s) are solids.

3. Use according to claim 1, wherein the multiphase system is a biphase system consisting of an oil phase/aqueous phase and the volume ratio non-aqueous phase(s) / aqueous phase ranges from 65: 35 to 20:80.

4. Use according to any one of claims 1 to 3, wherein the substituted polymeric saccharide is of formula (I) defined in claim 1 and the fructan-type saccharide is selected from the group consisting of inulin, oligofructose, fructo-oligosaccharide, partially hydrolysed inulin, levan, and partially hydrolysed levan.

5. Use according to claim 4, wherein the fructan-type saccharide is selected from the group consisting of polydisperse inulin from plant origin and oligofructose, fructo-oligosaccharide and partially hydrolysed inulin with a degree of polymerisation (DP) ranging from 3 to 9.

6. Use according to any one of claims 1 to 3, wherein the substituted polymeric saccharide is of formula (II) defined in claim 1 and the starch-type , saccharide is a starch hydrolysate with a dextrose equivalent (DE) ranging from 2 to 47.

7. Use according to any one of claims 1 to 6, wherein the hydrophobic moieties (-M) of the substituted polymeric saccharide of formula (I) or (II) as defined in claim 1 are all of the same nature, being alkylcarbamoyl radicals of formula R-NH-CO- wherein the alkyl radicals R can be the same or different and R has the meanings defined in claim 1.

8. Use according to any one of claims 1 to 6, wherein the hydrophobic moieties (-M) of the substituted polymeric saccharide of formula (I) or (II) as defined in claim 1 are all of the same nature, being alkylcarbonyl radicals of formula R-CO- wherein the alkyl radicals R can be the same or different and R has the meanings defined in claim 1.

9. Use according to claim 1, wherein the hydrophobic moieties (-M) of the substituted polymeric saccharide of formula (I) or (II) as defined in claim 1 are of a different nature being an alkylcarbamoyl radical of formula R-NH-CO- or an alkylcarbonyl radical of formula R-CO- , R having the meanings defined in claim 1 and wherein the alkyl radicals R are the same or are different.

10. Use according to claim 1 wherein the surfactant is composed of a mixture of two or more substituted polymeric saccharides of formula (I) and/or formula (II) as defined in any of the claims 7 to 9.

11. Use according to any one of claims 1 to 10 wherein the surfactant or mixture of surfactants is selected from the group consisting of compounds 1 to 31 indicated in the following table
| Product n° | Formula (I) or (II) | Type | Hydrophobic Moiety | | av.DS |
|---|---|---|---|---|---|
| | | | M | R- | |
| 1 | (I) | a | R-NH-CO | CH₃(CH₂)₇- | 0.02 |
| 2 | (I) | a | R-NH-CO | CH₃(CH₂)₇- | 0.08 |
| 3 | (I) | a | R-NH-CO | CH₃(CH₂)₇- | 0.09 |
| 4 | (I) | a | R-NH-CO | CH₃(CH₂)₇- | 0.2 |
| 5 | (I) | a | R-NH-CO | CH₃(CH₂)₁₁- | 0.07 |
| 6 | (I) | a | R-NH-CO | CH₃(CH₂)₁₁- | 0.08 |
| 7 | (I) | a | R-NH-CO | CH₃(CH₂)₁₁- | 0.1 |
| 8 | (I) | a | R-NH-CO | CH₃(CH₂)₁₁- | 0.1 |
| 9 | (I) | a | R-NH-CO | CH₃(CH₂)₁₁- | 0.1 |
| 10 | (I) | a | R-NH-CO | CH₃(CH₂)₁₁- | 0.12 |
| 11 | (I) | a | R-NH-CO | CH₃(CH₂)₁₁- | 0-15 |
| 12 | (I) | a | R-NH-CO | CH₃(CH₂)₁₁- | 0.21 |
| 13 | (I) | a | R-NH-CO | CH₃(CH₂)₁₁- | 0.3 |
| 14 | m | a | R-NH-CO | CH₃(CH₂)₁₅- | 0.21 |
| 15 | (I) | a | R-NH-CO | CH₃(CH₂)₁₇- | 0.023 |
| 16 | (I) | a | R-NH-CO | CH₃(CH₂)₁₇- | 0.054 |
| 17 | (I) | a | R-NH-CO | CH₃(CH₂)₁₇- | 0.11 |
| 18 | (I) | b | R-NH-CO | CH₃(CH₂)₁₁- | 0.3 |
| 19 | (I) | a | R-CO | CH₃(CH₂)₁₀- | 0.12 |
| 20 | (I) | a | R-CO | CH₃(CH₂)₁₄- | 0.1 |
| 21 | (I) | a | R-CO | CH₃(CH₂)₇CH=CH-(CH₂)₇ | 0.05 |
| 22 | (I) | a | R-CO | CH₃(CH₂)₁₆- | 0.11 |
| 23 | (II) | d | R-NH-CO | CH₃(CH₂)₁₁- | 0.05 |
| 24 | (II) | e | R-NH-CO | CH₃(CH₂)₁₁- | 0.1 |
| 25 | (II) | c | R-NH-CO | CH₃(CH₂)₁₁- | 0.1 |
| 28 | (II) | d | R-NH-CO | CH₃(CH₂)₁₁- | 0.18 |
| 27 | (II) | d | R-CO | CH₃(CH₂)₁₀- | 0.1 |
| 28 | (I) | a | R-NH-CO | CH₃(CH₂)₇- | 0.11 |
| 29 | (I) | a | R-NH-CO | CH₃(CH₂)₁₅- | 0.12 |
| 30 | (I) | f | R-NH-CO | CH₃(CH₂)₁₁- | 0.19 |
| 31 | m | f | R-NH-CO | CH₃(CH₂)₁₁- | 0.13 |
| Formula [A]ₙ(-M)ₛ (I) [B]ₘ(-M)_{s'} (II) | | | | | |
| a = inulin, av.DP:23 | | | | | |
| b = inulin, DP mainly between 2 and 8, av. DP: about 4.5 | | | | | |
| c = maltodextrin, DE 2 | | | | | |
| d = maltodextrin, DE 28 | | | | | |
| e = maltodextrin, DE 47 | | | | | |
| f = inulin, av. DP :13 | | | | | |

12. Use according to any one of claims 1 to 11, wherein the aqueous phase contains one or more electrolytes in a total concentration ranging from 0.5 moles/l to 5 moles/l.

13. Use according to claim 12, wherein the aqueous phase contains one or more electrolytes selected from the group consisting of metal salts, ammonium salts, amine salts, quaternary ammonium salts and salts of organic bases.

14. Use according to claim 13, wherein the salts are derived from hydrogen halides, sulphuric acid, phosphoric acid, carbonic acid and/ or lactic acid and/or are salts providing hydroxide anions when dissociating.

15. Use according to any of claims 1 to 14 wherein the dispersion additionally comprises one or more conventional surfactants, co-surfactants, thickeners, rheology modifiers and/or conventional additives.

16. Method for the preparation of a dispersion and/or for the stabilisation of a dispersion of a multiphase system that comprises a continuous aqueous phase containing one or more electrolytes at a total concentration ranging from the lower limit of 0.1 to 1 mole per litre, depending on the nature of the electrolyte(s) and the temperature of the dispersion, up to the limit of the solubility of the electrolyte(s) in water at 25°C, comprising bringing together and homogenising the composing phases of the multiphase system with addition of one or more hydrophobically modified saccharides of general formula (I) and/or (II) defined in claim 1 to the aqueous phase, or to the non-aqueous phase(s) or to the composing phases, whereby the total concentration of substituted polymeric saccharide or mixture of two or more substituted polymeric saccharides of formula (I) and/or formula (II) defined in claim 1 ranges from 0.10 to 20 %, being % w/v on dispersed phase(s) in case of emulsions, % w/w on dispersed phase(s) in case of suspensions, and % w/v on aqueous phase in case of foams, and optionally with addition of one or more conventional surfactants, co-surfactants and/or additives.

17. Dispersion of a multiphase system that comprises a continuous aqueous phase, **characterised in that** said aqueous phase contains one or more electrolytes at a total concentration ranging from the lower limit of 0.1 to 1 mole per litre aqueous phase, depending on the nature of the electrolyte(s) and the temperature of the dispersion, up to the limit of the solubility of the electrolyte(s) in water at 25°C, and that said dispersion comprises as surfactant one or more hydrophobically modified saccharides of general formula (I) or (II) defined in claim 1, whereby the total concentration of substituted polymeric saccharide or mixture of two or more substituted polymeric saccharides of formula (I) and/or formula (II) defined in claim 1 ranges from 0.10 to 20 %, being % w/v on dispersed phase(s) in case of emulsions, % w/w on dispersed phase(s) in case of suspensions, and % w/v on aqueous phase in case of foams, and optionally further comprises one or more conventional surfactants, co-surfactants and/ or additives.

18. Dispersion according to claim 17, wherein the multiphase system is a biphase system and said biphase system is an emulsion, a suspension or a foam.

## Patentansprüche

1. Verwendung eines Tensids zur Herstellung einer Dispersion eines Multiphasensystems, das eine kontinuierliche wässrige Phase umfasst, **dadurch gekennzeichnet, dass** die wässrige Phase ein oder mehrere Elektrolyte in einer Gesamtkonzentration im Bereich der Untergrenze von 0,1 bis 1 Mol pro Liter wässrige Phase, in Abhängigkeit von der Art des bzw. der Elektrolyte und der Temperatur der Dispersion, bis zur Löslichkeitsgrenze des bzw. der Elektrolyte in Wasser bei 25° C enthält und dass das Tensid ein hydrophob modifiziertes Saccharid ist, das ein substituiertes polymeres Saccharid mit der allgemeinen Formel (I) oder (II) ist,
[A]ₙ(-M)s (I) [B]ₘ(-M)_{s'} (II)
wobei
[A]ₙ ein fructanartiges Saccharid repräsentiert, in dem [A] eine Fructosyl-Einheit oder eine endständige Glucosyleinheit und n den Grad der Polymerisation (DP) darstellt, der die Anzahl an Fructosyl- und Glucosyleinheiten im Fructan-Saccharidmolekül ausdrückt.
[B]ₘ ein stärkeartiges Saccharid repräsentiert, in dem [B] eine Glucosyl-Einheit darstellt und m die Anzahl der Glucosyleinheiten im stärkeartigen Saccharidmolekül ausdrückt, das aus der Gruppe bestehend aus modifizierten Stärken und Stärkehydrolysaten mit einem Dextroseäquivalent (DE) im Bereich von 2 bis 47 ausgewählt ist,
(-M) einen hydrophoben Anteil repräsentiert, der ein Wasserstoffatom einer Hydroxylgruppe der Fructosyl- oder Glucosyleinheiten substituiert und der aus der Gruppe bestehend aus einem Alkylcarbamoylradikal mit der Formel R-NH-CO- und einem Alkylcarbonylradikal mit der Formel R-CO- ausgewählt ist, wobei R eine lineare oder verzweigte, gesättigte oder ungesättigte Alkylgruppe mit 4 bis 32 Kohlstoffatomen darstellt,
s und s', die denselben Wert haben können oder nicht, die Anzahl der hydrophoben Anteile repräsentieren, welche die Fructosyl- oder Glucosyleinheit substituieren, die als durchschnittlicher Substitutionsgrad (av. DS) im Bereich von 0,01 bis 0,5 ausgedrückt sind, und
die Gesamtkonzentration des substituierten, polymeren Saccharids mit der Formel (I) und/oder der Formel (II) im Bereich von 0,10 bis 20 % liegt, wobei % % Gew./Vol. bei einer dispergierten Phase bzw. Phasen im Falle von Emulsionen, % Gew./Gew. bei einer dispergierten Phase bzw. Phasen im Falle von Suspensionen und % Gew./Vol. bei wässrigen Phasen im Falle von Schäumen bedeutet.

2. Verwendung nach Anspruch 1, wobei das Multiphasensystem aus der Gruppe bestehend aus einem Biphasensystem und einem Triphasensystem ausgewählt ist, und das Verhältnis nicht wässrige Phase(n)wässrige Phase im Bereich von 90:10 bis 1:99 liegt, was als Volumen:Volumen-Verhältnis ausgedrückt ist, wenn die nicht wässrige(n) Phase(n) flüssige oder gasförmige Phasen sind, und als Gewicht:Volumen-Verhältnis ausgedrückt ist, wenn die nicht wässrige(n) Phase(n) Feststoffe sind.

3. Verwendung nach Anspruch 1, wobei das Multiphasensystem ein Biphasensystem bestehend aus einer Ölphase/wässrigen Phase und das Volumenverhältnis nicht wässrige Phase(n)/wässrige Phase im Bereich von 65:35 bis 20:80 liegt.

4. Verwendung nach jedem der Ansprüche 1 bis 3, wobei das substituierte, polymere Saccharid die in Anspruch 1 definierte Formel (I) aufweist, und das fructanartige Saccharid aus der Gruppe bestehend aus Inulin, Oligofructose, Fructooligosaccharid, teilweise hydrolysiertem Inulin, Levan und teilweise hydrolisiertem Levan ausgewählt ist.

5. Verwendung nach Anspruch 4, wobei das fructanartige Saccharid aus der Gruppe bestehend aus polydispersem Inulin pflanzlichen Ursprungs und Oligofructose, Fructooligosaccharid und teilweise hydrolysiertem Inulin mit einem Polymerisationsgrad (DP) im Bereich von 3 bis 9 ausgewählt ist.

6. Verwendung nach jedem der Ansprüche 1 bis 3, wobei das substituierte, polymere Saccharid die in Anspruch 1 definierte Formel (II) aufweist, und das stärkeartige Saccharid ein Stärkehydrolysat mit einem Dextroseäquivalent (DE) im Bereich von 2 bis 47 ist.

7. Verwendung nach jedem der Ansprüche 1 bis 6, wobei die hydrophoben Anteile (-M) des substituierten, polymeren Saccharids mit der Formel (I) oder (II), wie in Anspruch 1 definiert ist, alle derselben Art und Alkylcarbamoylradikale mit der Formel R-NH-CO- sind, wobei die Alkylradikale R gleichartig oder verschieden sein können und R die in Anspruch 1 definierten Bedeutungen hat.

8. Verwendung nach jedem der Ansprüche 1 bis 6, wobei die hydrophoben Teile (-M) des substituierten, polymeren Saccharids mit der Formel (I) oder (II), wie in Anspruch 1 definiert ist, alle derselben Art und Alkylcarbonylradikale mit der Formel R-CO- sind, wobei die Alkylradikale R gleich oder verschieden sein können, und R die in Anspruch 1 definierten Bedeutungen hat.

9. Verwendung nach Anspruch 1, wobei die hydrophoben Teile (-M) des substituierten, polymeren Saccharids mit der Formel (I) oder (II), wie in Anspruch 1 definiert ist, verschiedenartig sind, und ein Alkylcarbamoylradikal mit der Formel R-NH-CO- oder ein Alkylcarbonylradikal mit der Formel R-CO- sind, wobei R die in Anspruch 1 definierte Bedeutung hat und die Alkylradikale gleich oder verschieden sind.

10. Verwendung nach Anspruch 1, wobei das Tensid aus einer Mischung von zwei oder mehreren substituierten, polymeren Sacchariden mit der Formel (I) und/oder Formel (II) besteht, wie in jedem der Ansprüche 7 bis 9 definiert ist.

11. Verwendung nach jedem der Ansprüche 1 bis 10, wobei das Tensid oder die Mischung von Tensiden aus der Gruppe bestehend aus Verbindungen 1 bis 31, die in der folgenden Tabelle angeführt sind, ausgewählt ist.
| Produkt n° | Formel (I) oder (II) | Typ | Hydrophober Anteil | | Durchschn. DS |
|---|---|---|---|---|---|
| | | | M | R- | |
| 1 | (I) | a | R-NH-CO | CH₃(CH₂)₇- | 0,02 |
| 2 | (I) | a | R-NH-CO | CH₃(CH₂)₇- | 0,08 |
| 3 | (I) | a | R-NH-CO | CH₃(CH₂)₇- | 0,09 |
| 4 | (I) | a | R-NH-CO | CH₃(CH₂)₇- | 0,2 |
| 5 | (I) | a | R-NH-CO | CH₃(CH₂)₁₁- | 0,07 |
| 6 | (I) | a | R-NH-CO | CH₃(CH₂)₁₁- | 0,09 |
| 7 | (I) | a | R-NH-CO | CH₃(CH₂)₁₁- | 0,1 |
| 8 | (I) | a | R-NH-CO | CH₃(CH₂)₁₁- | 0,1 |
| 9 | (I) | a | R-NH-CO | CH₃(CH₂)₁₁- | 0,1 |
| 10 | (I) | a | R-NH-CO | CH₃(CH₂)₁₁- | 0,12 |
| 11 | (I) | a | R-NH-CO | CH₃(CH₂)₁₁- | 0,15 |
| 12 | (I) | a | R-NH-CO | CH₃(CH₂)₁₁- | 0,21 |
| 13 | (I) | a | R-NH-CO | CH₃(CH₂)₁₁- | 0,3 |
| 14 | (I) | a | R-NH-CO | CH₃(CH₂)₁₅- | 0,21 |
| 15 | (I) | a | R-NH-CO | CH₃(CH₂)₁₇- | 0,023 |
| 16 | (I) | a | R-NH-CO | CH₃(CH₂)₁₇- | 0,054 |
| 17 | (I) | a | R-NH-CO | CH₃(CH₂)₁₇- | 0,11 |
| 18 | (I) | b | R-NH-CO | CH₃(CH₂)₁₁- | 0,3 |
| 19 | (I) | a | R-CO | CH₃(CH₂)₁₀- | 0,12 |
| 20 | (I) | a | R-CO | CH₃(CH₂)₁₄- | 0,1 |
| 21 | (I) | a | R-CO | CH₃(CH₂)₇CH=C H-(CH₂)₇- | 0,05 |
| 22 | (I) | a | R-CO | CH₃(CH₂)₁₆- | 0,11 |
| 23 | (II) | d | R-NH-CO | CH₃(CH₂)₁₁- | 0,05 |
| 24 | (II) | e | R-NH-CO | CH₃(CH₂)₁₁- | 0,1 |
| 25 | (II) | c | R-NH-CO | CH₃(CH₂)₁₁- | 0,1 |
| 26 | (II) | d | R-NH-CO | CH₃(CH₂)₁₁- | 0,18 |
| 27 | (II) | d | R-CO | CH₃(CH₂)₁₀- | 0,1 |
| 28 | (I) | a | R-NH-CO | CH₃(CH₂)₇- | 0,11 |
| 29 | (I) | a | R-NH-CO | CH₃(CH₂)₁₅- | 0,12 |
| 30 | (I) | f | R-NH-CO | CH₃(CH₂)₁₁- | 0,18 |
| 31 | (I) | f | R-NH-CO | CH₃(CH₂)₁₁- | 013 |
| Formel [A]ₙ(-M)s (I) [B]ₘ(-M)ₛ' (II) | | | | | |
| a = Inulin, durchschnittl. DP: 23 | | | | | |
| b = Inulin, DP hauptsächlich zwischen 2 und 8, durchschnittl. DP: ca. 4.5 | | | | | |
| c = Maltodextrin, DE 2 | | | | | |
| d = Maltodextrin, DE 28 | | | | | |
| e = Maltodextrin, DE 47 | | | | | |
| f = Inulin, durchschnittl. DP:13 | | | | | |

12. Verwendung nach jedem der Ansprüche 1 bis 11, wobei die wässrige Phase ein Elektrolyt oder mehrere Elektrolyte in einer Gesamtkonzentration im Bereich von 0,5 Mol/l bis 5 Mol/l enthält.

13. Verwendung nach Anspruch 12, wobei die wässrige Phase ein oder mehrere Elektrolyte aus der Gruppe bestehend aus Metallsalzen, Ammoniumsalzen, Aminsalzen, quatemären Ammoniumsalzen und Salzen von organischen Basen enthält.

14. Verwendung nach Anspruch 13, wobei die Salze von Wasserstoffhaloiden, Schwefelsäure, Phosphorsäue, Kohlensäure und/oder von Milchsäure deriviert sind und/oder Salze sind, die Hydroxidanionen beim Dissoziieren bereitstellen.

15. Verwendung nach jedem der Ansprüche 1 bis 14, wobei die Dispersion zusätzlich ein oder mehrere konventionelle Tenside, Co-Tenside, Verdickungsstoffe, Rheologiemodifiziermittel und/oder konventionelle Additive enthält.

16. Verfahren zur Herstellung einer Dispersion und/oder zur Stabilisierung einer Dispersion eines Multiphasensystems, das eine kontinuierliche wässrige Phase umfasst, die ein oder mehrere Elektrolyte in einer Gesamtkonzentration im Bereich der Untergrenze von 0,1 bis 1 Mol pro Liter, in Abhängigkeit von der Art des bzw. der Elektrolyte und der Temperatur der Dispersion, bis zur Löslichkeitsgrenze des bzw. der Elektrolyte in Wasser bei 25° C enthält, welches das Zusammenbringen und Homogenisieren der zusammensetzenden Phasen des Multiphasensystems mittels Zugabe eines oder mehrerer hydrophob modifizierten Sacchariden mit der allgemeinen, in Anspruch 1 definierten Formel (I) und/oder (II) zur wässrigen Phase oder zur nicht wässrigen Phase(n) oder zu den zusammensetzenden Phasen, wobei die Gesamtkonzentration des substituierten polymeren Saccharids oder der Mischung aus zwei oder mehreren substituierten, polymeren Sacchariden der in Anspruch 1 definierten Formel (I) und/oder Formel (II) im Bereich von 0,10 bis 20 % liegt, wobei % % Gew./Vol. bei einer dispergierten Phase bzw. Phasen im Falle von Emulsionen, % Gew./Gew. bei einer dispergierten Phase bzw. Phasen im Falle von Suspensionen und % Gew./Vol. bei einer wässrigen Phase im Falle von Schäumen bedeutet, und wahlweise mittels Zugabe von einem oder mehreren konventionellen Tensiden, Co-Tensiden und/oder Additiven umfasst.

17. Dispersion eines Multiphasensystems, das eine kontinuierliche, wässrige Phase umfasst, **dadurch gekennzeichnet, dass** die wässrige Phase ein oder mehrere Elektrolyte in einer Gesamtkonzentration im Bereich der Untergrenze von 0,1 bis 1 Mol pro Liter wässriger Phase, in Abhängigkeit von der Art des Elektrolyts bzw. der Elektrolyte und der Temperatur der Dispersion, bis zur Löslichkeitsgrenze des Elektrolyts bzw. der Elektrolyte in Wasser bei 25° C enthält und dass die Dispersion als Tensid ein oder mehrere hydrophob modifizierte Saccharide mit der allgemeinen, in Anspruch 1 definierten Formel (I) oder (II) enthält, wobei die Gesamtkonzentration des substituierten polymeren Saccharids oder der Mischung aus zwei oder mehreren substituierten, polymeren Sacchariden der in Anspruch 1 definierten Formel (I) und/oder Formel (II) im Bereich von 0,10 bis 20 % liegt, wobei % % Gew./Vol. bei einer dispergierten Phase bzw. Phasen im Falle von Emulsionen, % Gew./Gew. bei einer dispergierten Phase bzw. Phasen im Falle von Suspensionen und % Gew.Nol. bei einer wässrigen Phase im Falle von Schäumen bedeutet, und wahlweise weiters ein oder mehrere konventionelle Tenside, Co-Tenside und/oder Additive enthält.

18. Dispersion nach Anspruch 17, wobei das Multiphasensystem ein Bisphasensystem ist und dieses Biphasensystem eine Emulsion. Suspension oder ein Schaum ist.

## Revendications

1. Utilisation d'un tensioactif pour la préparation d'une dispersion d'un système multiphasique qui comprend une phase aqueuse continue, **caractérisée en ce que** ladite phase aqueuse contient un ou plusieurs électrolytes à une concentration totale allant de la limite inférieure de 0,1 à 1 mole par litre de phase aqueuse, en fonction de la nature du ou des électrolytes et de la température de la dispersion, jusqu'à la limite de solubilité du ou des électrolytes dans de l'eau à 25°C, et **en ce que** ledit tensioactif est un saccharide à modification hydrophobe qui est un saccharide polymère substitué de formule générale (I) ou (II)
[A]ₙ(-M)ₛ (I)
[B]ₘ(-M)_{s'} (II)
dans laquelle
[A]ₙ représente un saccharide de type fructane, [A] représentant une unité fructosyle ou une unité glucosyle terminale et n représentant le degré de polymérisation (DP) qui est le nombre d'unités fructosyle et glucosyle dans ladite molécule de saccharide fructane,
[B]ₘ représente un saccharide de type amidon, [B] représentant une unité glucosyle et m étant le nombre d'unités glucosyle dans ladite molécule de saccharide de type amidon, choisie dans le groupe constitué par les amidons modifiés et les hydrolysats d'amidon avec un équivalent dextrose (DE) situé dans la plage allant de 2 à 47,
(-M) représente un groupement hydrophobe qui remplace un atome d'hydrogène d'un groupe hydroxyle desdits unités fructosyle ou glucosyle, qui est choisi dans le groupe constitué par un radical alkylcarbamoyle de formule R-NH-CO- et un radical alkylcarbonyle de formule R-CO-, où R représente un groupe alkyle linéaire ou ramifié, saturé ou insaturé, ayant de 4 à 32 atomes de carbone,
s et s', qui peuvent ou non avoir la même valeur, représentent le nombre desdits groupements hydrophobes qui substituent l'unité fructosyle ou glucosyle, exprimé en degré moyen de substitution (DS moy.) qui est situé dans la plage allant de 0,01 à 0,5, et
la concentration totale de saccharide polymère substitué de formule (I) et/ou de formule (II) est située dans la plage allant de 0,10 à 20 %, les pourcentages étant en poids/volume de la ou des phases dispersées dans le cas d'émulsions, en poids/poids de la ou des phases dispersées dans le cas de suspensions, et en poids/volume de la phase aqueuse dans le cas de mousses.

2. Utilisation selon la revendication 1, dans laquelle le système multiphasique est choisi dans le groupe constitué par un système biphasique et un système triphasique, et le rapport phase(s) non aqueuse(s)/phase aqueuse est situé dans la plage allant de 90/10 à 1/99, exprimée en rapport en volume/volume dans le cas où la ou les phases non aqueuses sont des phases liquides ou gazeuses, et en rapport en poids/volume dans le cas où la ou les phases non aqueuses sont des solides.

3. Utilisation selon la revendication 1, dans laquelle le système multiphasique est un système biphasique constitué d'une phase huileuse/phase aqueuse et le rapport en volume phase(s) non aqueuse(s)/phase aqueuse est situé dans la plage allant de 65/35 à 20/80.

4. Utilisation selon l'une quelconque des revendications 1 à 3, dans laquelle le saccharide polymère substitué est de formule (I) définie dans la revendication 1 et le saccharide de type fructane est choisi dans le groupe constitué par l'inuline, l'oligofructose, le fructo-oligosaccharide, l'inuline partiellement hydrolysée, la lévane, et la lévane partiellement hydrolysée.

5. Utilisation selon la revendication 4, dans laquelle le saccharide de type fructane est choisi dans le groupe constitué par l'inuline polydispersée d'origine végétale et l'oligofructose, le fructo-oligosaccharide et l'inuline partiellement hydrolysée ayant un degré de polymérisation (DP) situé dans la plage allant de 3 à 9.

6. Utilisation selon l'une quelconque des revendications 1 à 3, dans laquelle le saccharide polymère substitué est de formule (II) définie dans la revendication 1 et le saccharide de type amidon est un hydrolysat d'amidon ayant un équivalent dextrose (DE) situé dans la plage allant de 2 à 47.

7. Utilisation selon l'une quelconque des revendications 1 à 6, dans laquelle les groupements hydrophobes (-M) du saccharide polymère substitué de formule (I) ou (II) telle que définie dans la revendication 1 sont tous de même nature, étant des radicaux alkylcarbamoyle de formule R-NH-CO- où les radicaux alkyle R peuvent être identiques ou différents et R a les significations définies dans la revendication 1.

8. Utilisation selon l'une quelconque des revendications 1 à 6, dans laquelle les groupements hydrophobes (-M) du saccharide polymère substitué de formule (I) ou (II) telle que définie dans la revendication 1 sont tous de même nature, étant des radicaux alkylcarbonyle de formule R-CO- où les radicaux alkyle R peuvent être identiques ou différents et R a les significations définies dans la revendication 1.

9. Utilisation selon la revendication 1, dans laquelle les groupements hydrophobes (-M) du saccharide polymère substitué de formule (I) ou (II) telle que définie dans la revendication 1 sont de nature différente, étant un radical alkylcarbamoyle de formule R-NH-CO- ou un radical alkylcarbonyle de formule R-CO-, R ayant les significations définies dans la revendication 1, et où les radicaux alkyle R sont identiques ou différents.

10. Utilisation selon la revendication 1, dans laquelle le tensioactif est composé d'un mélange de deux, ou plusieurs, saccharides polymères substitués de formule (I) et/ou de formule (II) tel que défini selon l'une quelconque des revendications 7 à 9.

11. Utilisation selon l'une quelconque des revendications 1 à 10, dans laquelle le tensioactif ou le mélange de tensioactifs est choisi dans le groupe constitué par les composés 1 à 31 indiqués dans le tableau suivant :
| Produit N° | Formule (I) ou (II) | Type | Motif hydrophobe | | DS moy. |
|---|---|---|---|---|---|
| | | | M | R- | |
| 1 | (I) | a | R-NH-CO | CH₃(CH₂)₇- | 0,02 |
| 2 | (I) | a | R-NH-CO | CH₃(CH₂)₇- | 0,08 |
| 3 | (I) | a | R-NH-CO | CH₃(CH₂)₇- | 0,09 |
| 4 | (I) | a | R-NH-CO | CH₃(CH₂)₇- | 0,2 |
| 5 | (I) | a | R-NH-CO | CH₃(CH₂)₁₁- | 0,07 |
| 6 | (I) | a | R-NH-CO | CH₃(CH₂)₁₁- | 0,09 |
| 7 | (I) | a | R-NH-CO | CH₃(CH₂)₁₁- | 0,1 |
| 8 | (I) | a | R-NH-CO | CH₃(CH₂)₁₁- | 0,1 |
| 9 | (I) | a | R-NH-CO | CH₃(CH₂)₁₁- | 0,1 |
| 10 | (I) | a | R-NH-CO | CH₃(CH₂)₁₁- | 0,12 |
| 11 | (I) | a | R-NH-CO | CH₃(CH₂)₁₁- | 0,15 |
| 12 | (I) | a | R-NH-CO | CH₃(CH₂)₁₁- | 0,21 |
| 13 | (I) | a | R-NH-CO | CH₃(CH₂)₁₁- | 0,3 |
| 14 | (I) | a | R-NH-CO | CH₃(CH₂)₁₅- | 0,21 |
| 15 | (I) | a | R-NH-CO | CH₃(CH₂)₁₇- | 0,023 |
| 16 | (I) | a | R-NH-CO | CH₃(CH₂)₁₇- | 0,054 |
| 17 | (I) | a | R-NH-CO | CH₃(CH₂)₁₇- | 0,11 |
| 18 | (I) | b | R-NH-CO | CH₃(CH₂)₁₁- | 0,3 |
| 19 | (I) | a | R-CO | CH₃(CH₂)₁₀- | 0,12 |
| 20 | (I) | a | R-CO | CH₃(CH₂)₁₄- | 0,1 |
| 21 | (I) | a | R-CO | CH₃(CH₂)₇-CH=CH-(CH₂)₇ | 0,05 |
| 22 | (I) | a | R-CO | CH₃(CH₂)₁₆- | 0,11 |
| 23 | (II) | d | R-NH-CO | CH₃(CH₂)₁₁- | 0,05 |
| 24 | (II) | e | R-NH-CO | CH₃(CH₂)₁₁- | 0,1 |
| 25 | (II) | c | R-NH-CO | CH₃(CH₂)₁₁- | 0,1 |
| 26 | (II) | d | R-NH-CO | CH₃(CH₂)₁₁- | 0,18 |
| 27 | (II) | d | R-CO | CH₃(CH₂)₁₀ | 0,1 |
| 28 | (I) | a | R-NH-CO | CH₃(CH₂)₇- | 0,11 |
| 29 | (I) | a | R-NH-CO | CH₃(CH₂)₁₅- | 0,12 |
| 30 | (I) | f | R-NH-CO | CH₃(CH₂)₁₁- | 0,19 |
| 31 | (I) | f | R-NH-CO | CH₃(CH₂)₁₁- | 0,13 |
| Formules [A]ₙ(-M)ₛ (I) [B]ₘ(-M)_{s'} (II) | | | | | |
| a = inuline, DP moyen 23 | | | | | |
| b = inuline, DP principalement entre 2 et 8, DP moyen environ 4,5 | | | | | |
| c = maltodextrine, DE 2 | | | | | |
| d = maltodextrine, DE 28 | | | | | |
| e = maltodextrine, DE 47 | | | | | |
| f = inuline, DP moyen 13 | | | | | |

12. Utilisation selon l'une quelconque des revendications 1 à 11, dans laquelle la phase aqueuse contient un ou plusieurs électrolytes à une concentration totale située dans la plage allant de 0,5 mole/l à 5 moles/l.

13. Utilisation selon la revendication 12, dans laquelle la phase aqueuse contient un ou plusieurs électrolytes choisis dans le groupe constitué par les sels métalliques, les sels d'ammonium, les sels d'amine, les sels d'ammonium quaternaire et les sels de bases organiques.

14. Utilisation selon la revendication 13, dans laquelle les sels dérivent d'halogénures d'hydrogène, d'acide sulfurique, d'acide phosphorique, d'acide carbonique et/ou d'acide lactique et/ou sont des sels fournissant des anions hydroxyde lors d'une dissociation.

15. Utilisation selon l'une quelconque des revendications 1 à 14, dans laquelle la dispersion comprend de plus un ou plusieurs tensioactifs, co-tensioactifs, épaississants, agents modifiant la rhéologie conventionnels et/ou additifs conventionnels.

16. Procédé pour la préparation d'une dispersion et/ou pour la stabilisation d'une dispersion d'un système multiphasique qui comprend une phase aqueuse continue contenant un ou plusieurs électrolytes à une concentration totale allant de la limite inférieure de 0,1 à 1 mole par litre, en fonction de la nature du ou des électrolytes et de la température de la dispersion, jusqu'à la limite de solubilité du ou des électrolytes dans de l'eau à 25°C, comprenant l'étape consistant à mettre ensemble et homogénéiser les phases constitutives du système multiphasique avec addition d'un ou plusieurs saccharides à modification hydrophobe de formules générales (I) et/ou (II) définies dans la revendication 1 à la phase aqueuse, ou à ou aux phases non aqueuses ou aux phases constitutives, en conséquence de quoi la concentration totale de saccharide polymère substitué ou de mélange de deux ou plusieurs saccharides polymères substitués de formule (I) et/ou de formule (II) définies dans la revendication 1 est située dans la plage allant de 0,10 à 20 %, les pourcentages étant en poids/volume de la ou des phases dispersées dans le cas d'émulsions, en poids/poids de la ou des phases dispersées dans le cas de suspensions, et en poids/volume de la phase aqueuse dans le cas de mousses, et éventuellement avec addition d'un ou plusieurs tensioactifs, co-tensioactifs et/ou additifs conventionnels.

17. Dispersion d'un système multiphasique qui comprend une phase aqueuse continue, **caractérisée en ce que** ladite phase aqueuse contient un ou plusieurs électrolytes à une concentration totale allant de la limite inférieure de 0,1 à 1 mole par litre de phase aqueuse, en fonction de la nature du ou des électrolytes et de la température de la dispersion, jusqu'à la limite de solubilité du ou des électrolytes dans de l'eau à 25°C, et **en ce que** ladite dispersion comprend, en tant que tensioactif, un ou plusieurs saccharides à modification hydrophobe de formule générale (I) ou (II) définie dans la revendication 1, en conséquence de quoi la concentration totale de saccharide polymère substitué ou de mélange de deux ou plusieurs saccharides polymères substitués de formule (I) et/ou de formule (II) définies dans la revendication 1 est située dans la plage allant de 0,10 à 20 %, les pourcentages étant en poids/volume de la ou des phases dispersées dans le cas d'émulsions, en poids/poids de la ou des phases dispersées dans le cas de suspensions, et en poids/volume de la phase aqueuse dans le cas de mousses, et éventuellement comprend en outre un ou plusieurs tensioactifs, co-tensioactifs et/ou additifs conventionnels.

18. Dispersion selon la revendication 17, dans laquelle le système multiphasique est un système biphasique et ledit système biphasique est une émulsion, une suspension ou une mousse.
